# EUROPEAN PATENT APPLICATION

(11) **EP 2 264 463 A2**
(43) Date of publication of application: **22.12.2010**
(21) Application number: 10183974.4
(22) Date of filing: 18.03.2005
(51) Int. Cl.: G01N 33/68, G01N 33/74

(54) **Prognostic methods for congestive heart failure**

(30) Priority: 19.03.2004 US 554859 P
(62) Divisional of application: 05757019.4
(71) Applicant: Biomedica Medizinprodukte GmbH & Co KG, 1210 Wien (AT)
(72) Inventor: Ketelslegers, Jean-Marie, 1200 Bruxelles (BE); Rousseau, Michel, 1200 Bruxelles (BE)
(74) Representative: Sonn & Partner Patentanwälte

(57) **Abstract**

Provided are methods for predicting survival outcome of an individual suffering from congestive heart failure. In accordance with the method, a patient's survival outcome is determined by taking into consideration the levels of two markers in combination, the combination being more predictive of survival than either marker alone. The markers include endothelin, atrial natriurctic peptide and brain natriurctic peptide, their precursors and fragments thereof. Individuals with congestive heart failure identified as having increased risk of mortality using the methods of the invention are appropriately targeted for more aggressive therapy.

## Description

### FIELD OF THE INVENTION

The present invention relates to methods for determining prognosis of individuals suffering from congestive heart failure based on fluid levels of endothelin, atrial natriuretic peptide and other markers such as brain natriuretic peptide, their precursors and fragments thereof.

### BACKGROUND OF THE INVENTION

The following discussion of the background of the invention is merely provided to aid the reader in understanding the invention and is not admitted to describe or constitute prior art to the present invention.

Congestive heart failure (CHF) is characterized by the progressive activation of several endocrine systems (¹). Increased levels of natriuretic peptides as well as activation of the renin angiotensin-aldosterone system and of the sympathetic nervous system are associated with a poor prognosis (^{2;3}) Circulating plasma or serum levels of atrial natriuretic peptide (ANP) and brain natriuretic peptide (BNP), as well as of their N-terminal pro-peptides, have been extensively studied and found to be inversely related to the severity of left ventricular dysfunction and to be prognostic predictors (⁴⁻⁸). Also, BNP as well as N-proBNP have been recognized as prognostic markers of survival in several stages of cardiovascular failure. However, their relative prognostic potency in patients with severe pre-terminal CHF is not well delineated.

Similarly, endothelin-1 (ET-1) and the big endothelin-1 (Big ET-1) together with its fragments, have also been identified as significant prognostic factors (^{6;9-13}). Elevation of immunoreactive ET-1 levels in circulation was also observed in patients after myocardial infarction (¹⁴). However, data reporting the relative value of natriuretic peptides in comparison to ET-1 or big ET-1 remain scarce and conflicting. The factors that may contribute to the variability of the results include patient selection criteria, immunoassay techniques and reagents and the variables taken into account in the multivariate analysis.

The high mortality rate in patients with severe CHF and the complex issues to consider in selecting patients for heart transplant or aggressive bridge therapy increase the need for accurate prognostic markers. During the last two decades, improved outcomes with medical and surgical treatments have mitigated the assumed survival benefit of heart transplant over conventional therapy so that accurate selection of patients is becoming crucial (^{15;16}). In addition to the clinical evaluation and the selection criteria based on left ventricular function, exercise capacity, and hemodynamic data (^{17;18}), the assessment of neurohormonal markers could be valuable to better identify a CHF population with a poor prognosis.

### SUMMARY OF THE INVENTION

In one aspect, the present invention provides methods of predicting survival outcome for an individual suffering from congestive heart failure ("CHF"). The method uses two or more specified disease markers in combination to improve survival prediction (i.e., from death by cardiovascular cause) for many CHF patients. The combined application of two markers to a population of CHF patients, characterized by a common set of clinical parameters, allows the identification of a subgroup of patients with the worst survival prognosis, a subgroup of patients with the best survival prognosis, and a subgroup of patients with intermediate survival prognosis. In this respect, the combined use of the specified disease markers, is clinically more relevant for CHF patients stratification, than the use of each marker considered separately. Thus, the use of two (or more) markers in combination is more informative for outcome prognosis than either marker considered alone. In accordance with the method, survival prognosis may be improved for the high risk, intermediate risk and/or low risk categories.

In another aspect, the level of the first and second markers for an individual with CHF are compared to a cutoff level for each marker. A cutoff level for the first and second markers is chosen to distinguish between high and low survival rate from death by cardiovascular cause. In accordance with this method, the likelihood of death from cardiovascular cause is greatest when the two marker levels are greater than their cutoff levels, the likelihood of death from cardiovascular cause is least when the two marker levels are less than their cutoff levels, and the likelihood of death from cardiovascular cause is intermediate when one marker level is greater than the cutoff level and the other marker level is less than the cut off level. Thus, the method provides as the output a best risk, worst risk and intermediate risk grouping into which individual CHF patients fall.

In one embodiment, the individual has severe CHF and the first marker is Big ET-1 (1-38) or its immunologically detectable fragments while the second marker is N-proANP or its immunologically detectable fragments, proBNP or its immunologically detectable fragments, or ET-1 or its immunologically detectable fragments. In another embodiment, proBNP or its its immunologically detectable fragments is N-proBNP or its immunologically detectable fragments or BNP or its immunologically detectable fragments.

In another embodiment, the individual has severe CHF and the first marker is N-proBNP or its immunologically detectable fragments while the second marker is BNP or its immunologically detectable fragments.

In a further embodiment, the individual has mild to moderate CHF and the first marker is proANP or its immunologically detectable fragments while the second marker is proBNP or its immunologically detectable fragments or Big ET-1 (1-38) or its immunologically detectable fragments. In another embodiment, proBNP or its immunologically detectable fragments is N-proBNP or its immunologically detectable fragments or BNP or its immunologically detectable fragments.

In one embodiment, severe CHF is defined as New York Heart Association ("NYHA") class III-IV. In another embodiment, mild to moderate CHF is defined as NYHA class I-II.

In still further embodiments, different cutoff levels for various markers and marker fragments are provided. The cutoff levels may be represented as the value in units for the marker or as a fold the normal level (mean or median) for the marker.

In one embodiment involving severe CHF, the cutoff level for Big ET-1 (1-38) or its immunologically detectable fragments is about 2.4-5.0 fold the normal level of the marker. In another embodiment involving severe CHF, the cutoff level for Big ET-1 (1-38) is about 3.5-5.0 or more preferably about 4.1 fold the normal level of the marker. In still another embodiment involving severe CHF, the cutoff level for Big ET-1 (22-38) is about 2.4-2.6 or more preferably about 2.5 fold the normal level of the marker.

In another embodiment involving severe CHF, the cutoff level for proANP or its immunologically detectable fragments or N-proANP or its immunologically detectable fragments is about 3.3-12 fold the normal level of the marker. In yet another embodiment involving severe CHF, the cutoff level of proANP or its immunologically detectable fragments or N-proANP is about 3.3-4 or more preferably about 3.8 fold the normal level of the marker. In still another embodiment, involving severe CHF the cutoff level of N-proANP (1-25) is about 9.5-12 or more preferably about 10.7 fold the normal level of the marker. In still yet another embodiment involving severe CHF, the cutoff level of N-proANP (68-98) is about 8.5-10 or more preferably about 9.6 fold the normal level of the marker.

In another embodiment involving severe CHF, the cutoff level for proBNP or its immunologically detectable fragments is about 4.7-89 fold the normal level of the marker.

In another embodiment involving severe CHF, the cutoff level for N-proBNP its immunologically detectable fragments is about 4.7-6.8 or more preferably about 5.7 fold the normal level of the marker.

In another embodiment involving severe CHF, the cutoff level for BNP or its immunologically detectable fragments is about 16-89 fold the normal level of the marker. In yet another embodiment involving severe CHF, the cutoff level for BNP or its immunologically detectable fragments is 32-89 or more preferably about 54 fold the normal level of the marker. In still another embodiment involving severe CHF, the cutoff level for BNP or its immunologically detectable fragments is about 16-21 or more preferably about 18 fold the normal level of the marker.

In another embodiment involving severe CHF, the cutoff level for ET-1 or its immunologically detectable fragments is about 1.9-2.2 or more preferably about 2.1 fold the normal level of the marker.

In one embodiment involving mild to moderate CHF, the cutoff level for N-proANP or its immunologically detectable fragments is about 1.7-3.3 fold the normal level of the marker. In another embodiment involving mild to moderate CHF, the cutoff level of proANP or its immunologically detectable fragments or N-proANP (1-98) or its immunologically detectable fragments is about 1.7-2.2 or more preferably about 1.9 fold the normal level of the marker. In yet another embodiment involving mild to moderate CHF, the cutoff level for N-proANP (1-25) is about 2.6-3.3 or more preferably about 3 fold the normal level of the marker. In still another embodiment involving mild to moderate CHF, the cutoff level for N-proANP (68-98) is about 2.4-3.1 or more preferably about 2.8 fold the normal level of the marker.

In one embodiment involving mild to moderate CHF, the cutoff level for BNP or its immunologically detectable fragments is about 2.6-7 or more preferably about 4.2 fold the normal level of the marker. In another embodiment involving mild to moderate CHF, the cutoff level for BNP or its immunologically detectable fragments is about 4.4 -5.7 or yet more preferably about 5 fold the normal level of the marker.

In another embodiment involving mild to moderate CHF, the cutoff level for N-proANP (1-25) is about 2.6-3.3 or more preferably about 3 fold the normal level of the marker. In yet another embodiment involving mild to moderate CHF, the cutoff level for N-proANP (68-98) is about 2.4-3.1 or more preferably about 2.8 fold the normal level of the marker.

In another embodiment involving mild to moderate CHF, the cutoff level for N-proBNP or its immunologically detectable fragments is about 1.3-1.8 or more preferably about 1.5 fold the normal level of the marker.

In another embodiment involving mild to moderate CHF, the cutoff level for Big ET-1 (1-38) or its immunologically detectable fragments is about 1.6-2.4 or more preferably about 2 fold the normal level of the marker.

In a further aspect, the invention method provides percentage survival outcomes for the various risk groups.

In one embodiment involving severe CHF, when the level of Big ET-1 (1-38) or its immunologically detectable fragments and proANP or its immunologically detectable fragments or N-proANP or its immunologically detectable fragments are both less than the cutoff level, the individual's 50% survival outcome of death by cardiovascular cause is at least about 45-75 months or more likely at least about 91 months. In yet another embodiment involving severe CHF, when the level of Big ET-1 (1-38) or its immunologically detectable fragments and proANP or its immunologically detectable fragments or N-proANP or its immunologically detectable fragments are both more than the cutoff level, the individual's 50% survival outcome from death by cardiovascular cause is about 5 months.

In one embodiment involving severe CHF, when the level of Big ET-1 (1-38) or its immunologically detectable fragments and proBNP or its immunologically detectable fragments are both less than the cutoff level, the individual's 50% survival for death by cardiovascular cause is at least about 61 months. In yet another embodiment involving severe CHF, when the level of Big ET-1 (1-38) or its immunologically detectable fragments and proBNP or its immunologically detectable fragments are both more than the cutoff level, the 50% survival outcome for the individual is about 4.5-7.5 months.

In one embodiment involving severe CHF, when the level of Big ET-1 (1-38) or its immunologically detectable fragments and N-proBNP or its immunologically detectable fragments are both less than the cutoff level, the individual's 50% survival for death by cardiovascular cause is at least about 61 months. In yet another embodiment involving severe CHF, when the level of Big ET-1 (1-38) or its immunologically detectable fragments and N-proBNP or its immunologically detectable fragments are both more than the cutoff level, the 50% survival outcome for the individual is about 7.5 months.

In another embodiment involving severe CHF, when the level of Big ET-1 (1-38) or its immunologically detectable fragments and BNP or its immunologically detectable fragments are both less than the cutoff level, the 50% survival outcome for the individual is about 31-44 months. In yet another embodiment involving severe CHF, when the level of Big ET-1 (1-38) or its immunologically detectable fragments and BNP or its immunologically detectable fragments are both more than the cutoff level, the individual's 50% survival outcome for death by cardiovascular cause is about 4.5 months.

In another embodiment involving severe CHF, when the level Big ET-1 (1-38) or its immunologically detectable fragments and ET-1 or its immunologically detectable fragments are both less than the cutoff level, the individual's 50% survival outcome for death by cardiovascular cause is about 37 months. In yet another embodiment involving severe CHF, when said level of Big ET-1 (1-38) or its immunologically detectable fragments and ET-1 or its immunologically detectable fragments are both more than the cutoff level, the individual's 50% survival outcome for death by cardiovascular cause is about 4.5 months.

In another embodiment involving severe CHF, when the level of N-proBNP or its immunologically detectable fragments and BNP or its immunologically detectable fragments are both less than the cutoff level, the individual's 50% survival outcome for death by cardiovascular cause is about 44 months. In yet another embodiment involving severe CHF, when the level of N-proBNP or its immunologically detectable fragments and BNP or its immunologically detectable fragments are both more than the cutoff level, the individual's 50% survival outcome for death by cardiovascular cause is about 5 months.

In one embodiment involving mild to moderate CHF, when the level of proANP or its immunologically detectable fragments or N-proANP or its immunologically detectable fragments and proBNP or its immunologically detectable fragments are both less than the cutoff level, the individual's 75% survival outcome for death by cardiovascular cause is at least about 91 months. In yet another embodiment involving mild to moderate CHF, when the level of proANP or its immunologically detectable fragments or N-proANP or its immunologically detectable fragments and proBNP or its immunologically detectable fragments are both more than the cutoff level, the individual's 75% survival outcome for death by cardiovascular cause is about 35-42 months.

In one embodiment involving mild to moderate CHF, when the level of proANP or its immunologically detectable fragments or N-proANP or its immunologically detectable fragments and BNP or its immunologically detectable fragments are both less than the cutoff level, the individual's 75% survival outcome for death by cardiovascular cause is at least about 91 months. In yet another embodiment involving mild to moderate CHF, when the level of proANP or its immunologically detectable fragments or N-proANP or its immunologically detectable fragments and BNP or its immunologically detectable fragments are both more than the cutoff level, the individual's 75% survival outcome for death by cardiovascular cause is about 35-42 months.

In another embodiment involving mild to moderate CHF, when the level of proANP or its immunologically detectable fragments or N-proANP or its immunologically detectable fragments and N-proBNP or its immunologically detectable fragments are both less than the cutoff level, the individual's 75% survival outcome for death by cardiovascular cause is at least about 91 months. In yet another embodiment involving mild to moderate CHF, when the level of proANP or its immunologically detectable fragments or N-proANP or its immunologically detectable fragments and N-proBNP or its immunologically detectable fragments are both more than the cutoff level, the individual's 75% survival outcome for death by cardiovascular cause is about 40 months.

In another embodiment involving mild to moderate CHF, when the level of proANP or its immunologically detectable fragments or N-proANP or its immunologically detectable fragments and Big ET-1 or its immunologically detectable fragments are both less than the cutoff level, the individual's 75% survival outcome for death by cardiovascular cause is at least about 91 months. In yet another embodiment involving mild to moderate CHF, when the level of proANP or its immunologically detectable fragments or N-proANP or its immunologically detectable fragments and Big ET-1 or its immunologically detectable fragments are both more than the cutoff level, the individual's 75% survival outcome for death by cardiovascular cause is about 40 months.

### BRIEF DESCRIPTION OF THE DRAWINGS

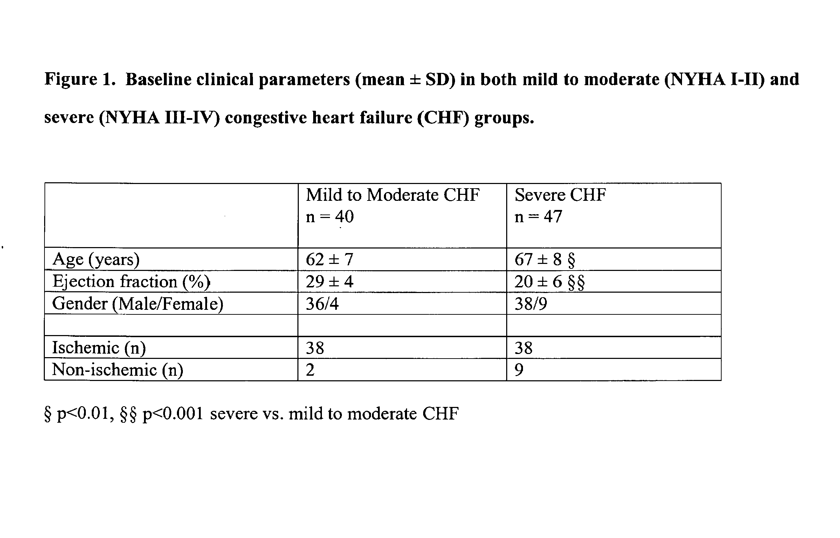

FIG. 1 is a Table showing baseline clinical parameters in both mild to moderate and severe CHF groups.

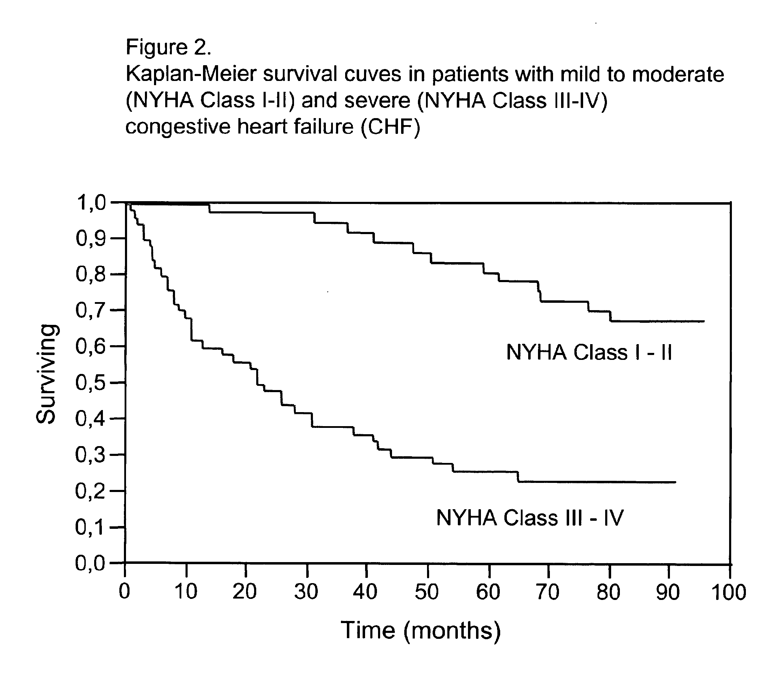

FIG. 2 shows Kaplan-Meier survival curves in patients with Congestive Heart Failure (CHF) stratified into 2 groups according to the severity of their disease: mild to moderate CHF (New York Heart Association class I-II) and severe CHF (New York Heart Association -NYHA- class III-IV).

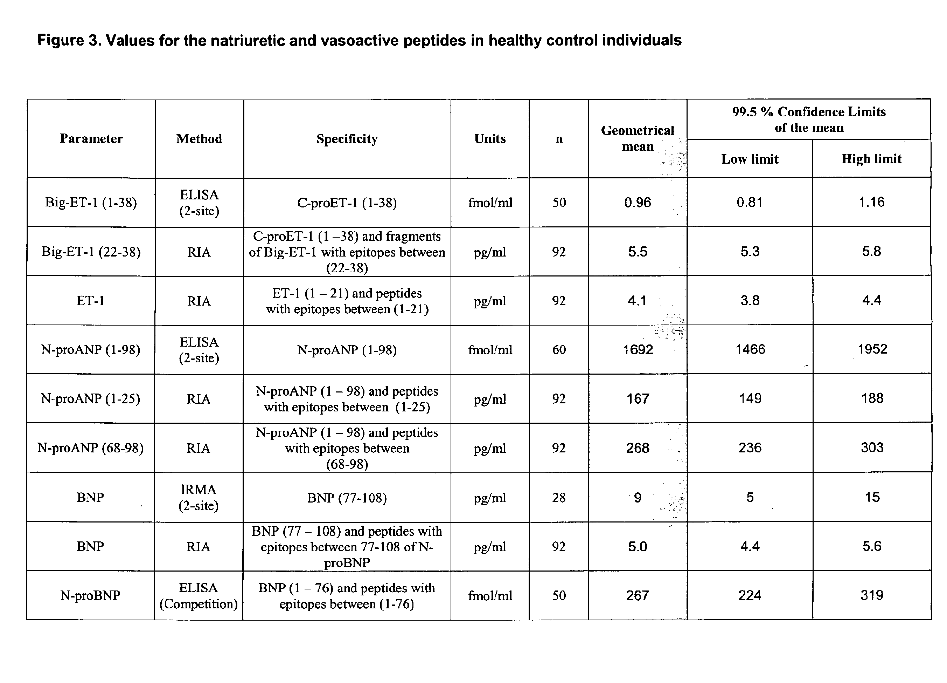

FIG. 3 is a Table showing the normal values for neurohormonal parameters (natriuretic and vasoactive peptides), in healthy control individuals.

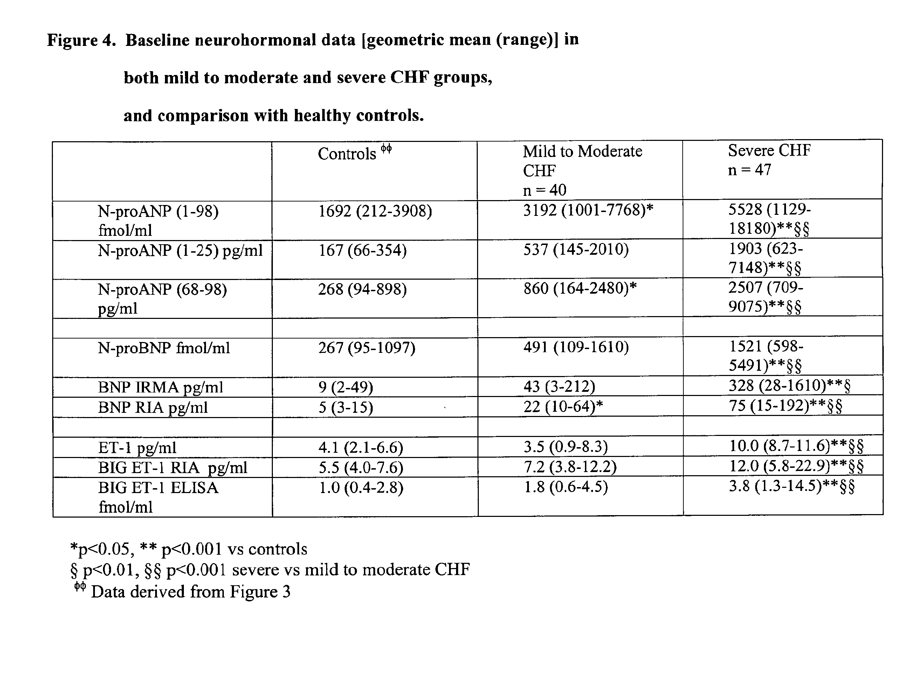

FIG. 4 is a Table showing the baseline neurohormonal data in both mild to moderate and severe congestive heart failure (CHF) groups and comparison with healthy controls.

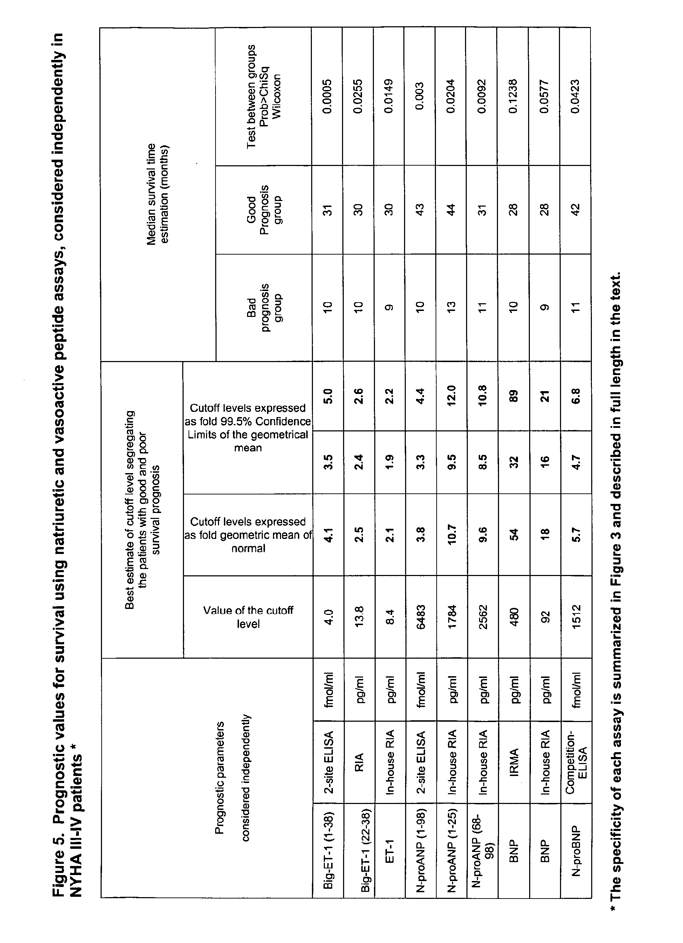

FIG. 5 is a Table showing the prognostic values for survival using natriuretic and vasoactive peptide assays, considered individually in patients with severe CHF (New York Heart Association -NYHA- Class III-IV).

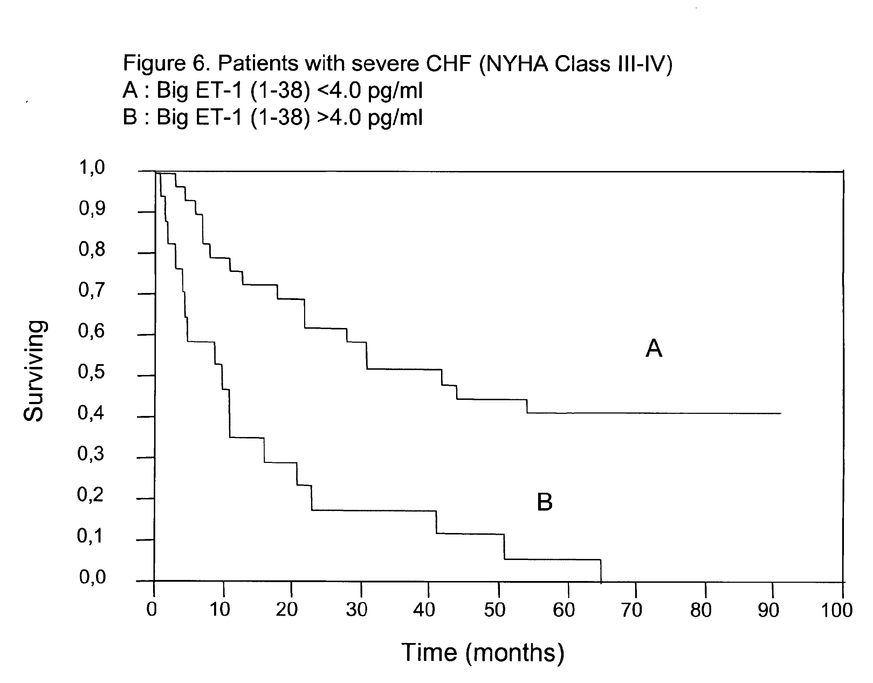

FIG. 6 shows Kaplan-Meier survival curves in patients with severe congestive heart failure (New York Heart Association class III-IV) stratified in two groups according to plasma levels of Big ET-1 (1-38) lower than 4.0 pg/ml (A) and higher than 4.0 pg/ml (B).). The survival parameters of both groups are in the table in FIG. 5

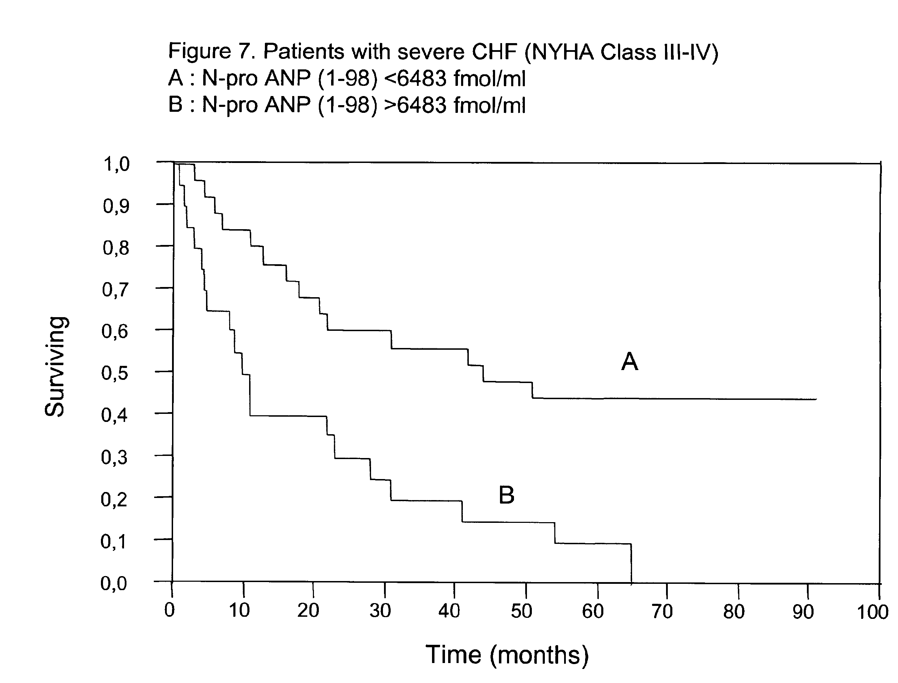

FIG. 7 shows Kaplan-Meier survival curves in patients with severe CHF (New York Heart Association class III-IV), stratified in two groups according to plasma levels of N-proANP (1-98) lower than 6483 fmoles/ml (A), and higher than 6483 fmoles/ml (B). The survival parameters of both groups are in the table in FIG. 5

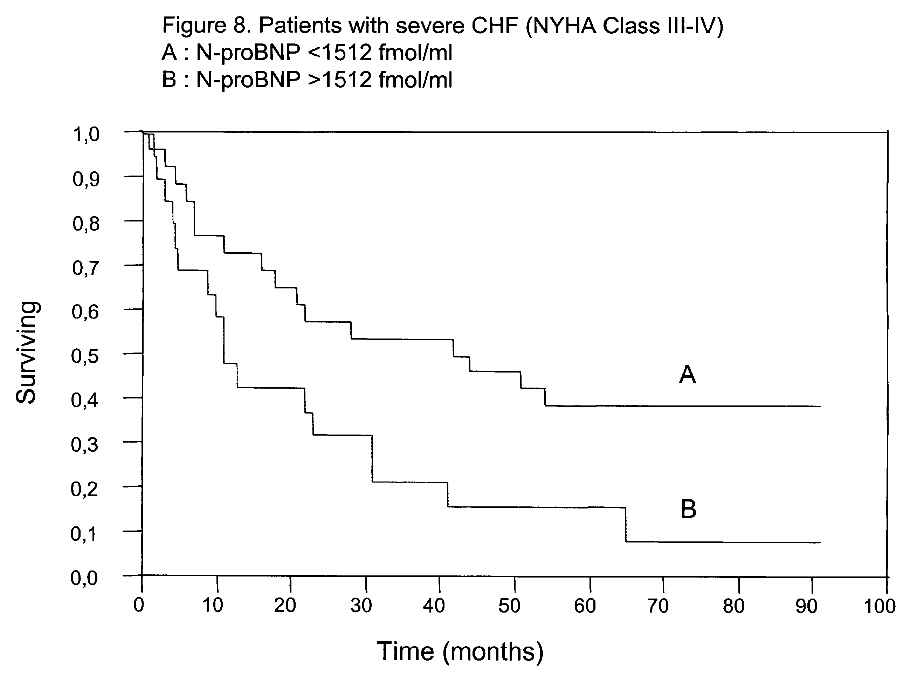

FIG. 8 shows Kaplan-Meier survival curves in patients with severe CHF (New York Heart Association class III-IV), stratified according to plasma levels ofN-proBNP. Patients are stratified in two groups according to plasma levels of N-proBNP lower than 1512 fmoles/ml (A) and higher than 1512 fmoles/ml (B). The survival parameters of both groups are in the table in FIG. 5

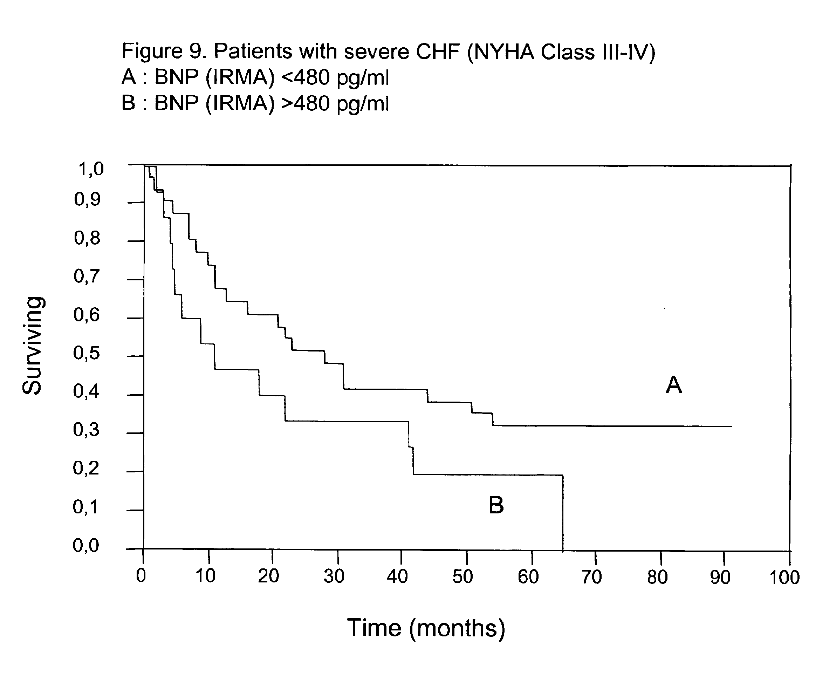

FIG. 9 shows Kaplan-Meier survival curves in patients with severe CHF (New York Heart Association class III-IV), stratified according to plasma levels of BNP measured with IRMA. Patients are stratified in two groups according to plasma levels of BNP lower than 480 pg/ml (A), and higher than 480 pg/ml (B). The survival parameters of both groups are in the Table in FIG. 5

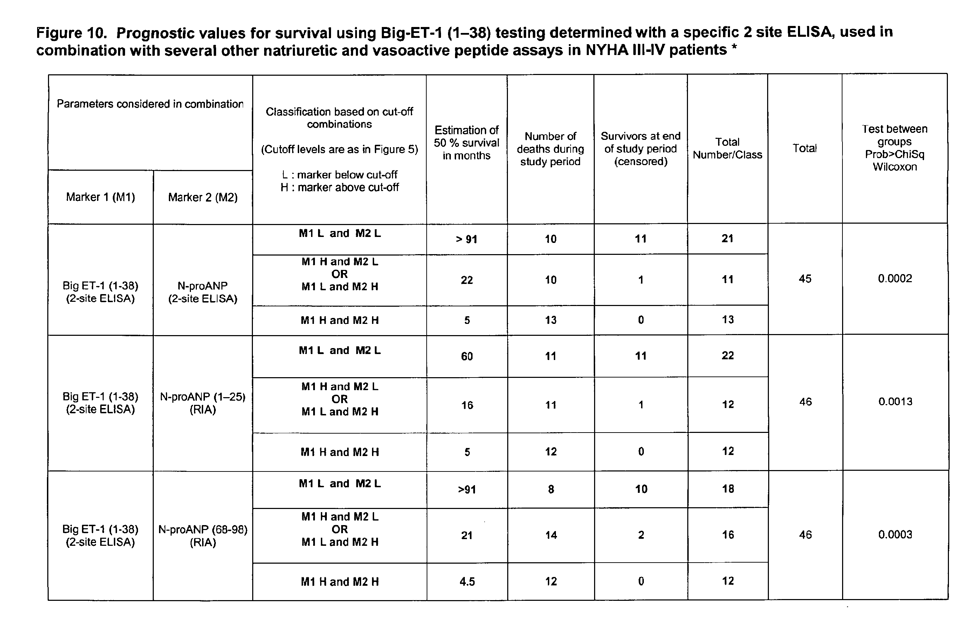

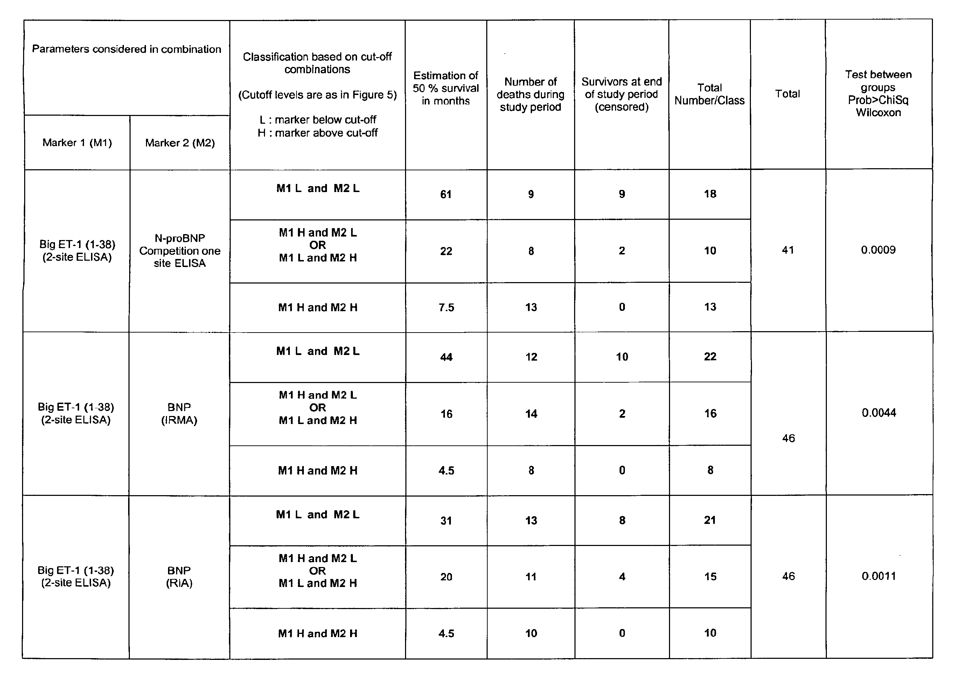

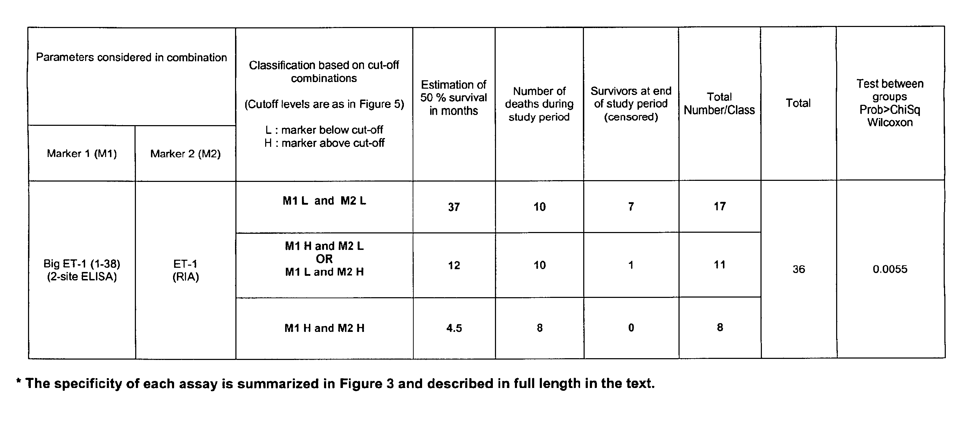

FIG. 10 is a Table showing the prognostic values for surviving death by cardiovascular cause using Big ET-1 (1-38) testing, used in combination with several other natriuretic and vasoactive peptides in NYHA III - IV patients.

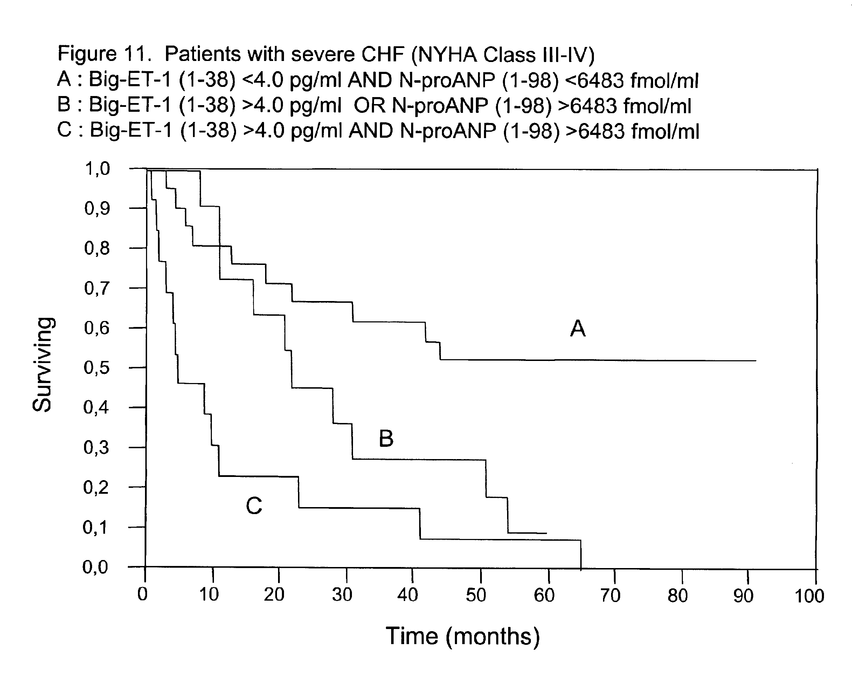

FIG. 11 shows Kaplan-Meier survival curves in patients with severe CHF (New York Heart Association -NYHA- Class III-IV), stratified according to plasma levels of Big ET-1 (1-38) in combination with N-proANP (1-98). Cut-off values used are as in FIG. 5. Patients are stratified into 3 groups: both parameters lower than their respective cut-off values (A); patients with one of the parameters above their cut-off, but the other below their cut-off (B); patients with both parameters above their cut-off values (C). The survival parameters of the three groups are in the table in FIG. 10.

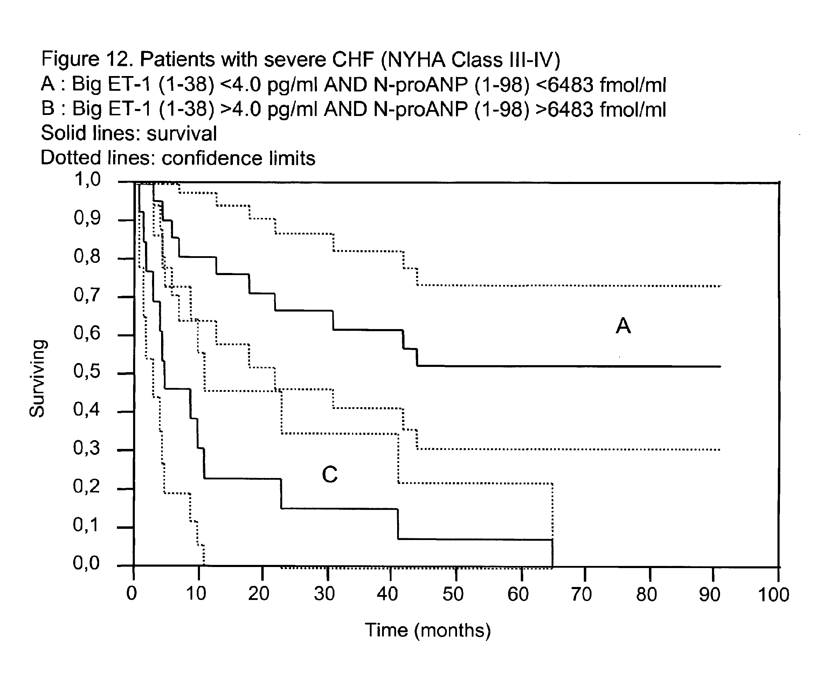

FIG. 12 shows the Kaplan-Meier survival curves in patients with severe CHF (New York Heart Association -NYHA- Class III-IV), stratified according to plasma levels of Big ET-1 (1-38), in combination with N-proANP (1-98). Only patients with both parameters below (A) or above (C) their respective cut-offs are represented. The survival curves are represented with their confidence intervals (dotted lines).

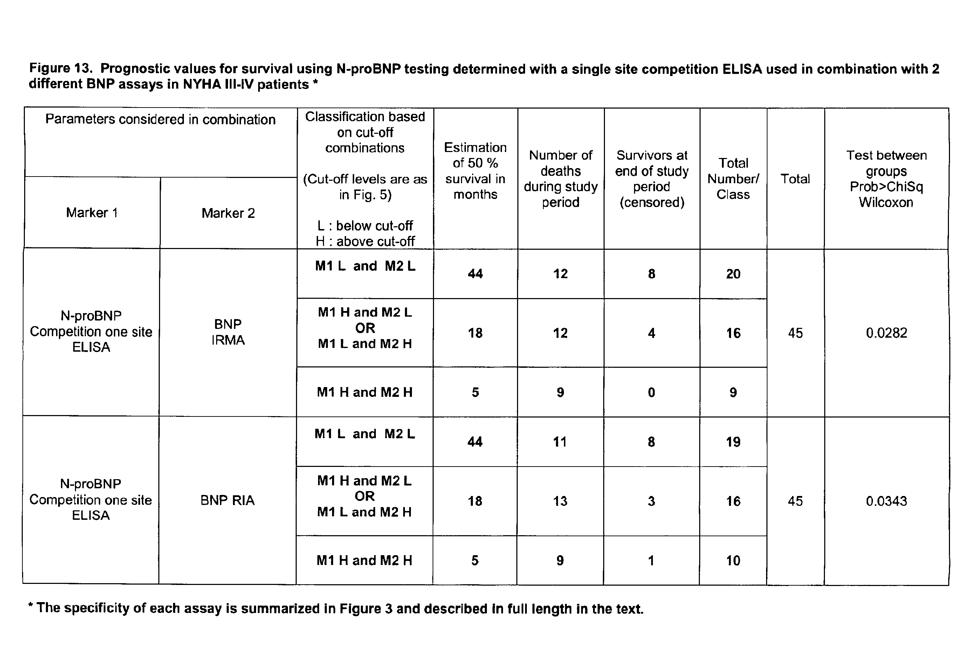

FIG. 13 is a Table showing the prognostic values for survival using N-proBNP testing, used in combination with BNP testing, determined by IRMA or by RIA, in patients with severe CHF (NYHA Class III-IV patients).

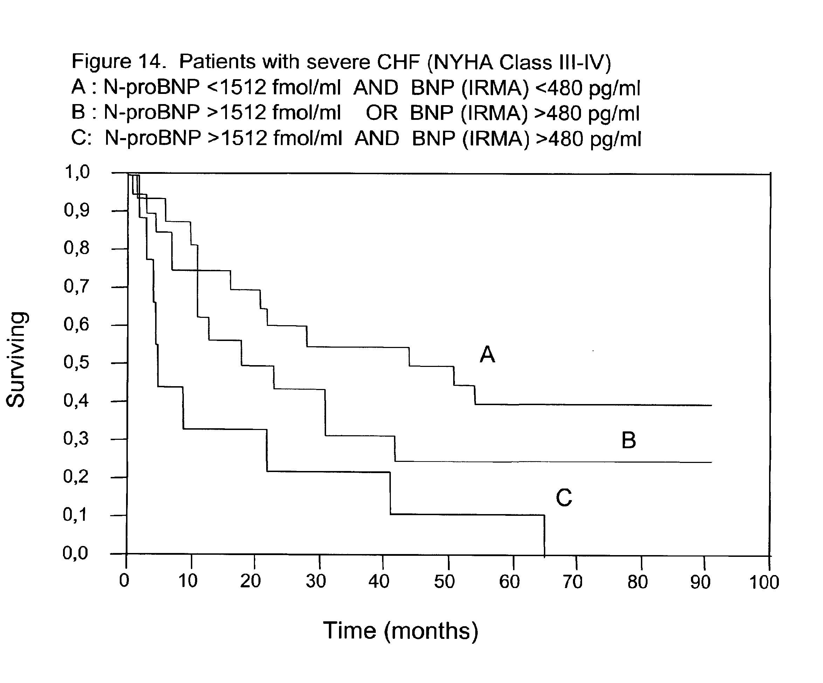

FIG. 14 shows the Kaplan Meier survival curves in patients with severe CHF (New York Heart Association class III-IV), stratified according to plasma levels N-proBNP, in combination with plasma BNP measured with IRMA. Cut-off values used are derived from FIG 5, FIG. 8 and FIG 9. Patients are stratified into 3 groups: both parameters lower that their respective cut-off values (A); patients with one of the parameters above their cut-off, but the other below their cut-off (B); patients with both parameters above their cut-off values (C). The survival parameters of the three groups are in the table in FIG. 13.

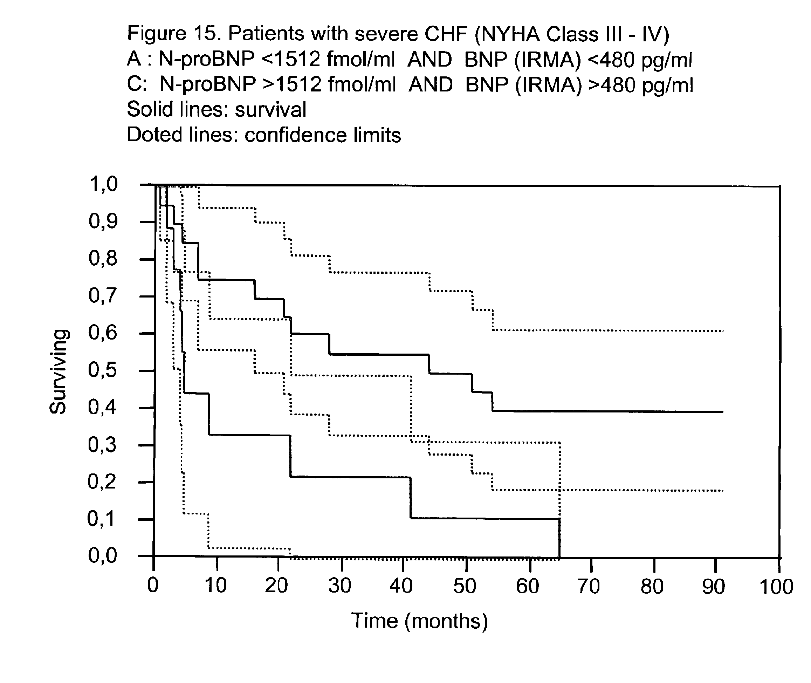

FIG. 15 shows the Kaplan-Meier survival curves in patients with severe CHF (New York Heart Association class III-IV), stratified according to plasma levels N-proBNP, in combination with BNP testing determined with a IRMA. Cut-off values used are derived from FIG 5, FIG. 8 and FIG 9. Only patients with both parameters below (A) or above (C) their respective cut-offs are represented. The survival curves are represented with their confidence intervals (dotted lines).

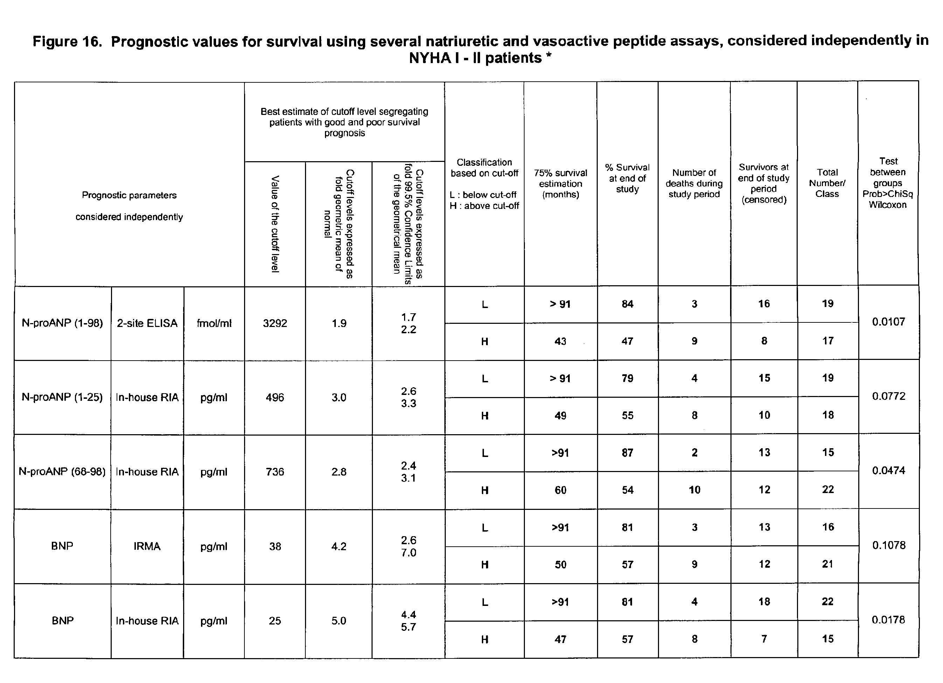

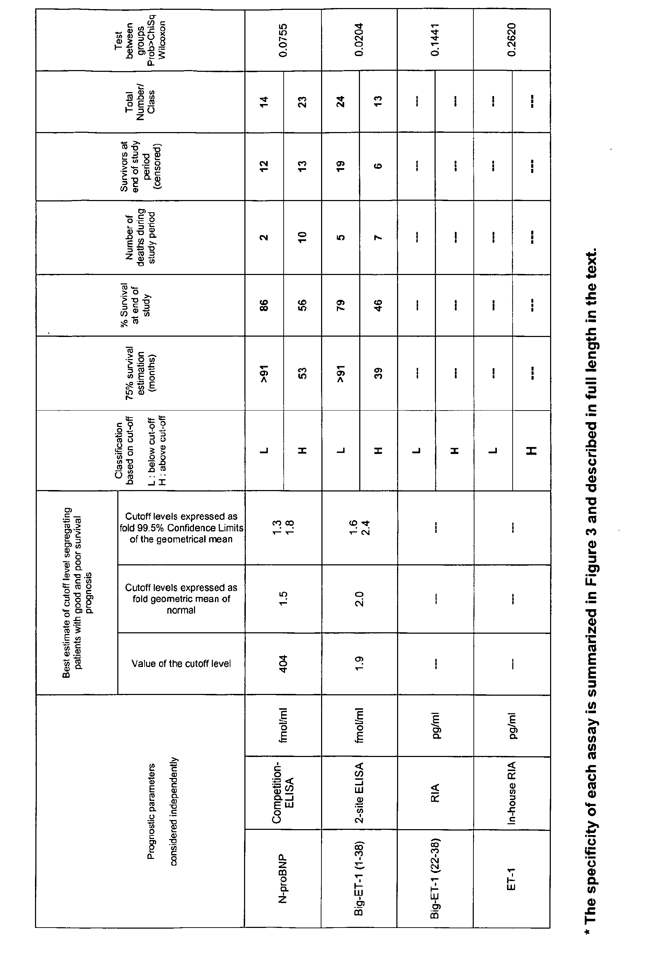

FIG. 16 is a table showing the prognostic values for survival using several natriuretic and vasoactive peptide assays, considered individually in patients with mild to moderate CHF (New York Heart Association -NYHA- Class I-II).

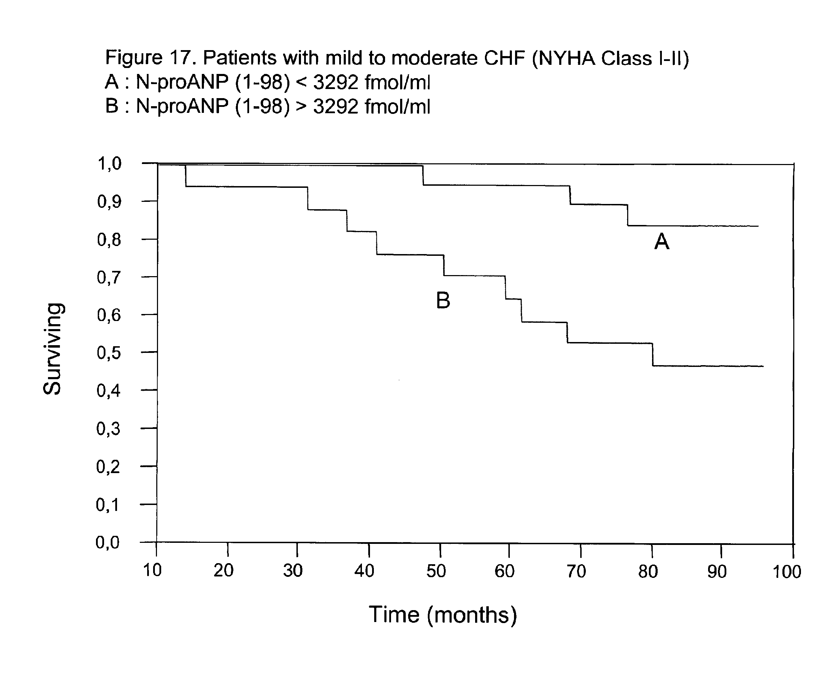

FIG. 17 shows Kaplan-Meier survival curves in patients with mild to moderate CHF (New York Heart Association class I-II), stratified in two groups according to plasma levels of N-proANP (1-98) lower than 3292 fmoles/ml (A) and higher than 3292 fmoles/ml (B). The survival parameters of both groups are in the table in FIG. 16.

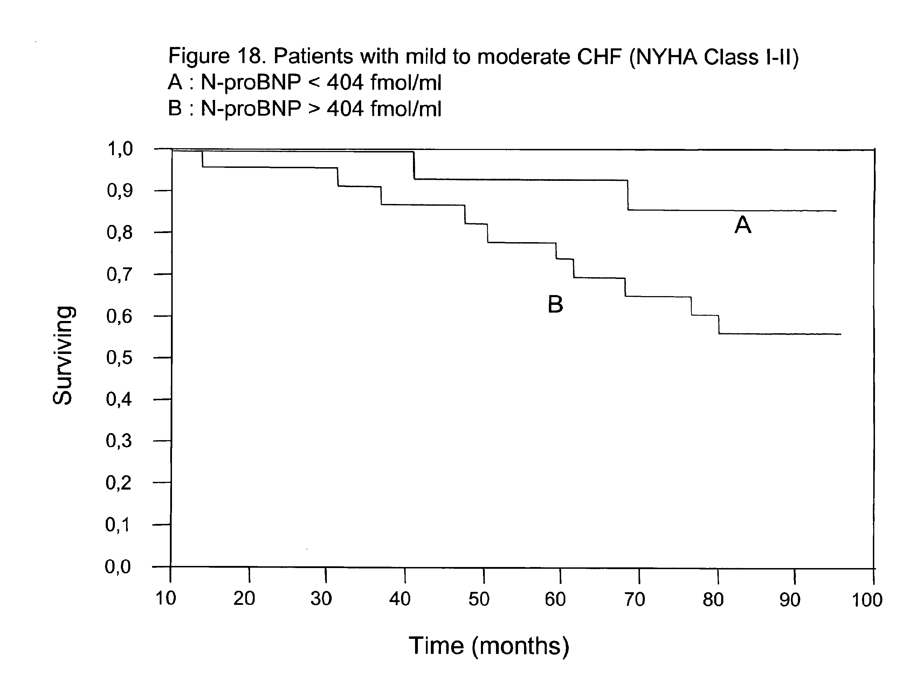

FIG. 18 shows Kaplan-Meier survival curves in patients with mild to moderate CHF (New York Heart Association class I-II), stratified in two groups according to plasma levels ofN-proBNP lower than 404 fmoles/ml (A) and higher than 404 fmoles/ml (B). The survival parameters of both groups are in the table in FIG. 16.

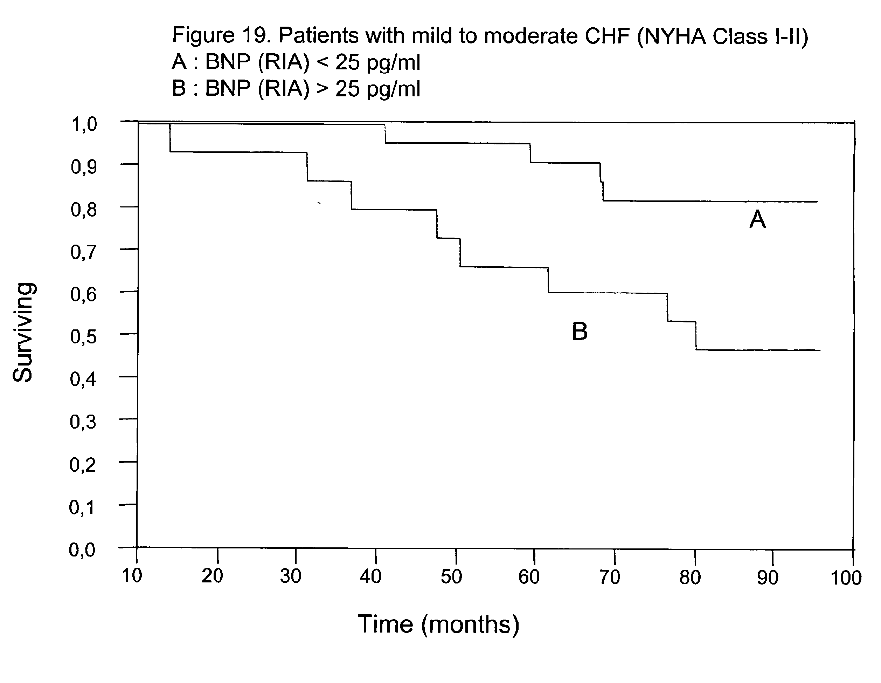

FIG. 19 shows Kaplan-Meier survival curves in patients with mild to moderate CHF (New York Heart Association class I-II), stratified in two groups according to plasma levels of BNP determined by RIA lower than 25 pg/ml (A) and higher than 25 pg/ml (B). The survival parameters of both groups are in the table in FIG. 16.

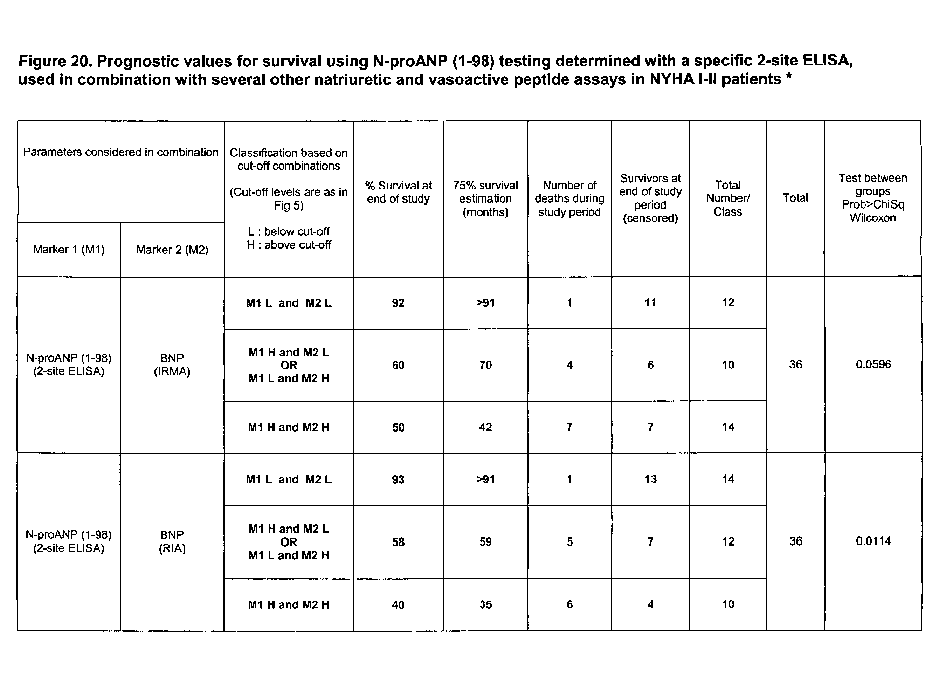

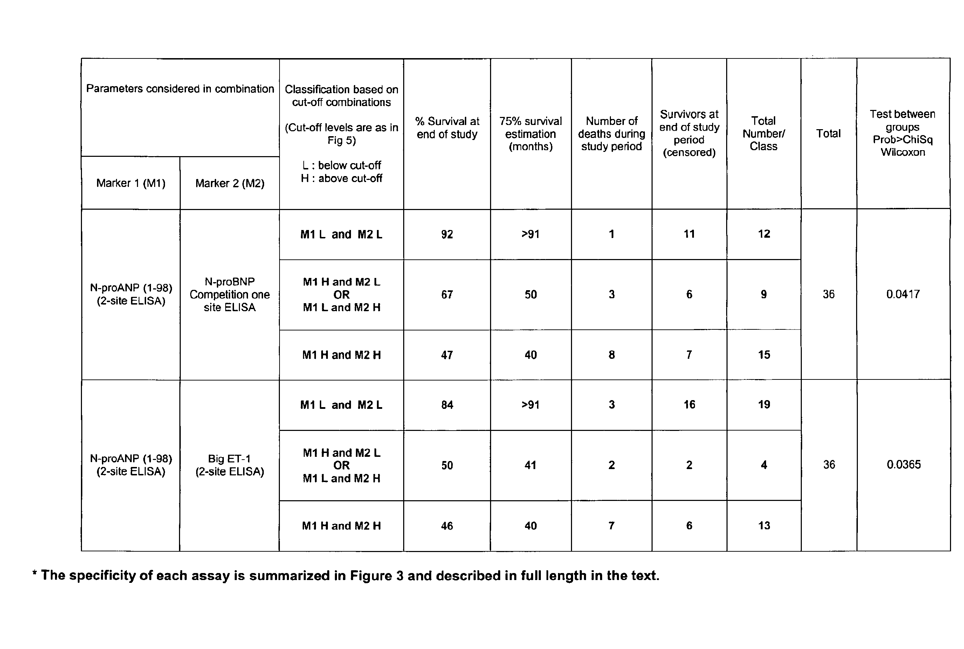

FIG 20 is a table showing the prognostic values for survival using N-proANP (1-98) testing used in combination with several other natriuretic and vasoactive peptide assays in patients with mild to moderate CHF (New York Heart Association -NYHA- Class I-II).

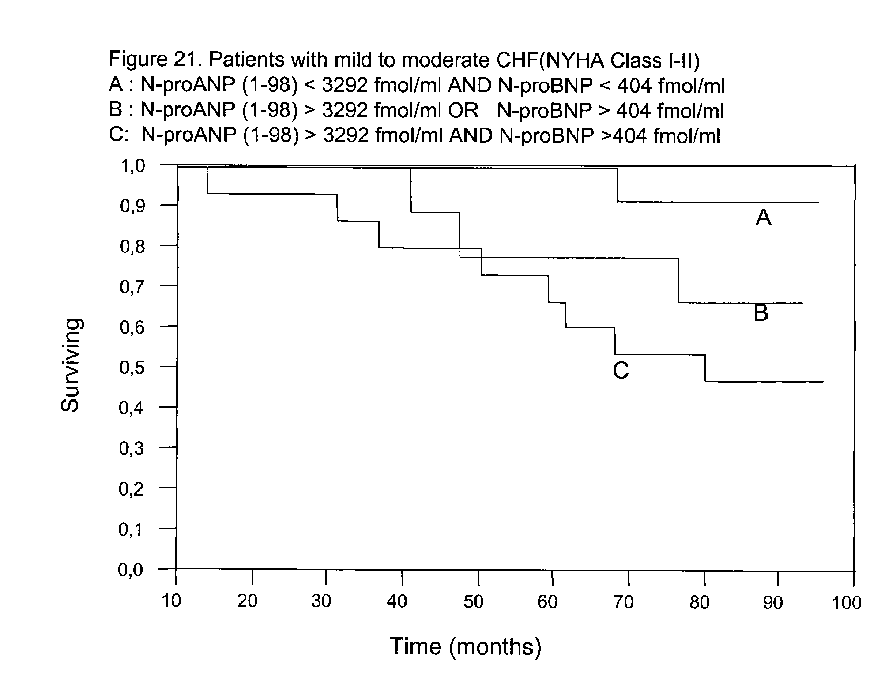

FIG. 21 shows Kaplan-Meier survival curves in patients with mild to moderate CHF (New York Heart Association -NYHA- Class I-II), stratified according to plasma levels of N-proANP (1-98) in combination with plasma N-proBNP. Cut-off values used are as in FIGs. 17 and 18. Patients are stratified into 3 groups: both parameters lower than their respective cut-off values (A); patients with one of the parameters above their cut-off, but the other below their cut-off (B); patients with both parameters above their cut-off values (C). The survival parameters of the three groups are in the Table in FIG. 20.

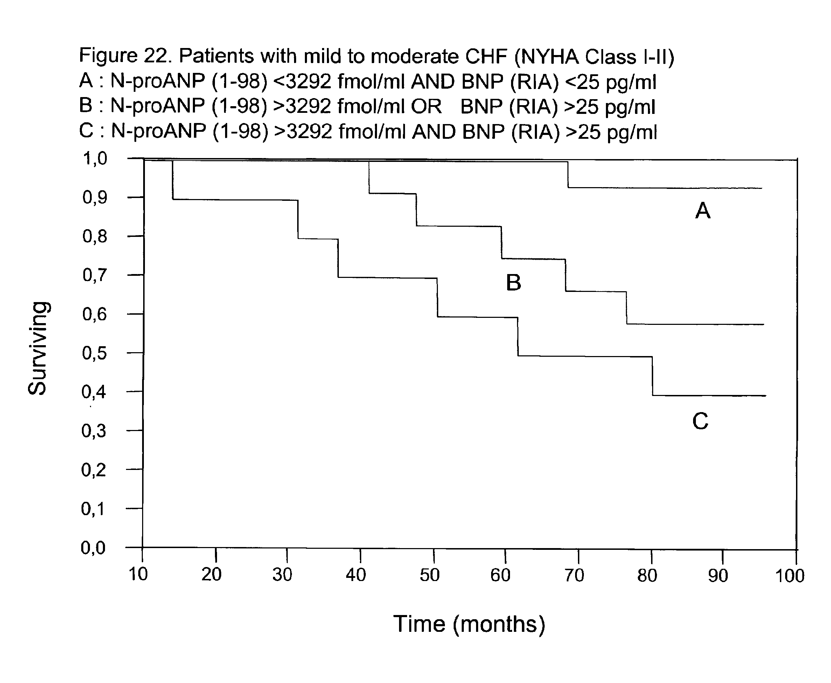

FIG. 22 shows Kaplan-Meier survival curves in patients with mild to moderate CHF (New York Heart Association -NYHA- Class I-II), stratified according to plasma levels of N-proANP (1-98) in combination with plasma BNP determined by RIA. Cut-off values used are as in FIGs. 17 and 19. Patients are stratified into 3 groups: both parameters lower than their respective cut-off values (A); patients with one of their parameters above the cut-off, but the other below their cut-off (B); patients with both parameters above their cut-off values (C). The survival parameters of both groups are in the Table in FIG. 20.

### DETAILED DESCRIPTION OF THE INVENTION

The present invention provides methods of predicting survival outcome of an individual suffering from severe or mild to moderate congestive heart failure using at least two disease markers that are more predictive in combination than either marker alone. In accordance with one aspect of the invention, the levels of the specified markers are determined and compared to a cutoff value representing high versus low risk for each marker. Higher risk is present when the levels of both markers in the individual are above their cutoff; and lower risk is present when the levels of both markers in the individual are below their cutoff. Intermediate risk is present when one marker level in the individual is above their cutoff and the other marker level is below their cutoff.

The term "Big endothelin-1 (1-38)" ("Big ET-1 (1-38) or "Big ET-1") as used herein refers to a peptide of 38 amino acids, which normally contains two disulfide bonds. Big ET-1 (1-38) is generated by enzymatic cleavage of an approximately 200 amino acid precursor called prepro-endothelin (preproET). "Big- ET-1 (1-38)" is not active and represents positions 53-90 of preproET (¹⁹).

The term Big-endothelin (22-38) ("Big ET-1 (22-38)") (also known as C-terminal fragment or CTF of Big ET-1 (1-38)) as used herein refers to the 17 amino acids at the carboxyl-terminal end of "Big ET-1 (1-38)." Big ET-1 (22-38) results from proteolytic cleavage of "Big ET-1 (1-38)" at position Trp⁷³ - Val⁷⁴ of preoproET- via the action of endothelin-converting enzyme (²⁰). This corresponds to positions Trp 21 - Val²² of "Big ET-1", when the first amino acid of "Big ET-1" is numbered beginning at position 1.

The term endothelin-1 ("ET-1") as used herein refers to the 21 amino acid amino-terminal fragment Big ET-1 (1-38), which normally contains the 2 disulfide bonds (^{21;22}). ET-1 is produced by proteolytic cleavage via the action of endothelin-converting enzyme, which cleaves Big ET-1 (1-38) at position Trp²¹ - Val²² to yield the 21-residue amino-terminal fragment. ET-1 has multifunctional properties including venous and arterial vasoconstriction, ability to modulate cardiac inotropy and to induce cardiomyocytes hypertrophy and gene expression (²³⁻²⁶). Plasma ET-1 concentrations represent spillover from local tissue production, which is dependent on the presence, but also of the cleavage rate of Big ET-1, and thus of the activation of the endothelin-convening enzyme. ET-1 circulating levels are also affected by rapid blood clearance, particularly in the lungs, probably through the ET-B receptors (^{9;27-29})_{.}

The term "immunologically detectable fragments" as used herein refers to fragments of a specified polypeptide (or ("parent" polypeptide") that can be detected immunologically. Such fragments have at least one epitope which is shared with the parent polypeptide and/or have at least one epitope that is absent from or poorly detectable in the parent polypeptide but, nevertheless, constitutes an identifying characteristic of the fragment. Thus, immunologically detectable fragments have at least one epitope that is associated with or derived from the parent polypeptide. The term "immunologically detectable" implies that the characterizing epitope(s) of the fragment are relatively specific to the fragment so that other polypeptides or fragments will not be appreciably detected. For example, immunologically detectable fragments of Big ET-1 (1-38) include those which can be detected by immunological means and which are do not include other polypeptides and their fragments in circulation (e.g., the natriuretic peptides). Although a fragment is "immunologically detectable" it may be detected by other methods known in the art including, chromatography, HPLC, mass spectrometry, and the like.

The term "Big ET-1 or its immunologically detectable fragments" as used herein refers to Big ET-1 (1-38), Big ET-1 (22-38), Big ET-1 (1-38) truncated at its carboxyl-terminal or amino-terminal end by one or more amino acids, more preferably one to ten amino acids (e.g. Big ET-1 (1-31) or (1-32) (³⁰), and fragments with at least one epitope that is associated with or derived from Big ET-1. However, the term "Big ET-1 or its immunologically detectable fragments" does not include ET-1 or its immunologically detectable fragments"

The term "ET-1 or its immunologically detectable fragments" as used herein refers to ET-1, fragments thereof truncated at its amino- or carboxyl-terminal ends by one or more amino acids, more preferably one to six amino acids, and fragments with at least one epitope that is associated with or derived from the ET-1.

The circulating levels of Big ET-1 or its immunologically detectable fragments can be determined by RIA or competitive non-isotopic immunoassays. Exemplary assays are described in the Examples. Specifically, Big ET-1 (22-38) together with Big ET-1 (1-38) can be determined by RIA or non-isotopic immunoassays. The circulating levels of Big ET-1 (1-38) also can be determined by a 2-site ("sandwich") immunoassay.

The term "pro-atrial natriuretic peptide" ("proANP (1-126)"or "proANP") as used herein refers to the 126 amino acid peptide product resulting from cleavage of the amino-terminal signal sequence from the 151 amino acids preproANP peptide. PreproANP is the transcriptional product of the preproANP mRNA (³¹⁻³⁴).

The term, Atrial Natriuretic Peptide ("proANP (99-126)" or "ANP") as used herein, refers to the 28 amino acid carboxyl-terminal fragment of proANP(1-126). ANP is the biologically active natriuretic fragment of the ANP precursor (³⁵⁻³⁸).

The term, "N-pro-atrial natriuretic (1 - 98)" peptide ("N-proANP (1-98)" or "N-proANP") as used herein, refers to the first 98 amino acids from the amino-terminal end of proANP (1-126), enzymatically cleaved from proANP.

The term N-proANP (1 - 25) as used herein refers to the first 25 amino acids from the amino-terminal end of N-proANP.

The term N-proANP (68 - 98) as used herein refers to the last 31 last amino acids at the carboxy-terminal end of N-proANP.

The term "N-proANP or its immunologically detectable fragments" as used herein refers to N-proANP (1-98), N-proANP (1 - 25), N-proANP (68 - 98), and fragments of N-proANP truncated at the amino- or carboxyl- terminal ends by one or more amino acids, more preferably one to 35 amino acids, (e.g., ANP (31- 67) or related fragments (³⁹)), and fragments with at least one epitope that is associated with or derived from N-proANP.

The term "proANP or its immunologically detectable fragments" as used herein refers to the full length 126 amino acid ANP precursor, fragments thereof containing the entire sequence or part of the sequence of ANP, N-proANP (1-98), N-proANP (1 - 25), N-proANP (68 - 98), ANP (99-126), and fragments of proANP truncated at the amino- or carboxyl- terminal ends by one or more amino acids, more preferably, one to 35 amino acids and fragments with at least one epitope that is associated with or derived from proANP.

The circulating levels of N-proANP or its immunologically detectable fragments can be determined by RIAs or non-isotopic immunoassays in accordance with well know methods. Exemplary assays are described in the Examples. Specifically, N-proANP (1-98) can be determined by a 2-site ("sandwich") immunoassay. The levels of full-length N-proANP (1-98) and fragments thereof presenting the N-proANP (1-25) or the N-proANP (68-98) epitopes as well as the intermediate fragments such as ANP (31-67) can be determined by standard RIAs. Immunologically detectable fragments of N-proANP can be detected with immunological reagents that do not cross-react appreciably with ANP or its immunologically detectable fragments.

The term "pro-brain natriuretic peptide" ("proBNP (1-108)" or "proBNP") refers to the 108 amino acid peptide product resulting from cleavage of the amino-terminal signal sequence from the preproANP peptide, which is the transcriptional product of preproBNP mRNA (⁴⁰)_{.}

The term "brain natriuretic peptide" ("BNP (77-108)" or "BNP") as used herein, refers to the 32 amino acid carboxyl-terminal fragment of proBNP(1-108). BNP is the biologically active natriuretic fragment of the BNP precursor (^{41;42})

The term "BNP or its immunologically detectable fragments" as used herein, refers to the 32 amino acid carboxyl-terminal fragment of proBNP(1-108) truncated at the amino or carboxy terminal end by one or more amino acids, more preferably one to 7 amino acids, and fragments with at least one epitope that is associated with or derived from BNP.

The term, "N-pro-brain natriuretic (1 - 76)" peptide ("N-proBNP (1-76)" or "N-proBNP") as used herein, refers to the first 76 amino acids located on the amino-terminal end of proBNP (1-108), enzymatically cleaved from proBNP.

The term "N-proBNP or its immunologically detectable fragments" as used herein refers to N-proBNP (1-76) and fragments of N-proBNP truncated at the amino- or carboxyl-terminal ends by one or more amino acids, more preferably one to 20 amino acids, and fragments with at least one epitope that is associated with or derived from N-proBNP. Immunologically detectable fragments of N-proBNP can be detected with immunological reagents that do not cross-react appreciably with BNP or its immunologically detectable fragments.

The term "proBNP or its immunologically detectable fragments" as used herein refers to the full length 108 amino acid BNP precursor, fragments thereof containing the entire sequence or part of the sequence of BNP, N-proBNP (1-76), BNP (77-108), and fragments of proBNP truncated at the amino- or carboxyl- terminal ends by one or more amino acids, more preferably, one to 35 amino acids and fragments with at least one epitope that is associated with or derived from proANP.

The levels of circulating N-proBNP and BNP or their fragments can be determined by RIA, IRMA or by non-isotopic assays. Exemplary assays are described in the Examples. Specifically, BNP can be measured by a 2-site ("sandwich") IRMA or RIA. N-proBNP can be measured by a competitive non-isotopic assay.

The term "determining a level of a (first or second) marker" as used herein refers to using an assay to measure the concentration of an analyte in the circulation of an individual. The level of the analyte in circulation may be the serum or plasma concentration of the analyte. FIG. 3 lists various assays that can be used to measure levels of the markers as a concentration such as in fmol/ml, pg/ml, and the like. The level of the markers in circulation of normal individuals and in severe CHF or mild to moderate CHF is provided in FIG. 7.

The term "cutoff level" as used herein refers to the level of a marker, which provides an optimal separation between high and low survival rate in severe CHF or mild to moderate CHF. In practice, a cutoff value is determined for each marker by trial and error iterations, the goal being an optimal classification of the patients into a group with poor prognosis and a group with better survival prognosis. The survival curves of the groups with values above and below a given cut-off value can be estimated using the Kaplan-Meier method, while the significance of the difference between groups, classified on the basis of each cutoff value, can be estimated using the log-rank and the Wilcoxon tests. All statistical analysis was performed using JMP software package release 5.0.1.2, running under Windows NT v5.0, (SAS Institute Inc.).

In one embodiment, cutoff levels for various markers in severe CHF are given FIG. 5 under the heading "value of the cutoff level." In one embodiment, cutoff levels for various markers in mild to moderate CHF are given in FIG. 16 under the heading "value of the cutoff level." In another embodiment, the cutoff level for severe CHF and mild to moderate CHF is "about" the values shown in the respective figures. The term "about" as used herein means plus or minus 5%.

The term "the normal level of the marker" as used in connection with the cutoff level of a marker refers to the cutoff level expressed as the fold (mean or median) of the level value of the marker in controls. In a preferred embodiment, the mean is the geometric mean. In a preferred embodiment, the control is the marker level in an age matched group of healthy individuals. In a preferred embodiment, the cutoff level for markers in severe CHF can be expressed as the fold geometric mean and expressed as the fold 99.5% confidence limit. Exemplary values are given in FIG. 5. In a preferred embodiment, the cutoff level for markers in mild to moderate CHF can be expressed as the fold geometric mean and expressed as the fold 99.5% confidence limits. Exemplary values are shown in FIG. 16. In a further embodiment, the cutoff level as the fold geometric mean or as the fold 99.5% confidence limits in both severe CHF and mild to moderate CHF is "about" the values shown in the respective figures. Preferably, the cutoff value and the normal value for the marker which are used to make the ratio are obtained with the same or similar assay.

The term, "left ventricular ejection fraction" (LVEF) as used herein, refers to the fraction of blood present in the left ventricle at the end of the diastolic phase, which is ejected in the circulation at the end of the systolic phase.

The term, "congestive heart failure" (CHF) as used herein, refers to "the pathophysiologic state in which an abnormality of the cardiac function is responsible for the failure of the heart to pump blood at a rate commensurate with the requirements of the metabolizing tissues and/or can do so only from an abnormally elevated diastolic volume (⁴³).

The term "New York Heart Association (NYHA) Classification" is a functional classification to assess cardiovascular disease. The system always referred to as NYHA CHF Classification is in widespread use. The former NYHA is now part of the American Heart Association, Inc. The general features of the classification are as follows:
# Class I: patients with no limitation of activities; they suffer no symptoms from ordinary activities.
# Class II: patients with slight, mild limitation of activity; they are comfortable with rest or with mild exertion.
# Class III: patients with marked limitation of activity; they are comfortable only at rest.
# Class IV: patients who should be at complete rest, confined to bed or chair; any physical activity brings on discomfort and symptoms occur at rest.

The term, "severe CHF as used herein, refers to patients with NYHA III or IV class.

The term, "mild CHF" or "mild to moderate CHF" as used herein, refers to patients with NYHA I or II class.

The term "death by cardiovascular cause" as used herein refers to a sudden death such as fatal arythmia and to progressive forms of cardiac failure such as heart pump failure, and the like. Patients with CHF dying from non-cardiovascular causes such as cancer, automobile accident, and the like, are not included within the meaning of death by cardiovascular cause.

The term "radioimmunoassay" (RIA) as used herein refers to a competitive immunoassay, based on the use of a polyclonal antiserum (or monoclonal antibody) and a tracer peptide labeled with a radioactive probe (usually radioactive iodine).

The term, "immunoradiometric assay" (IRMA) as used herein, refers to a 2-site ("sandwich") assay, in which the analyte to be measured is captured by an antibody linked to a solid phase and detected by another antibody labeled with a radioactive probe (usually radioactive iodine).

The term "Enzyme linked immunosorbent assay" (ELISA) as used herein refers to a competitive or 2-site ("sandwich") assay in which the tracer peptide or detection antibody is labeled with an enzyme detected by a colorimetric reaction.

The term "predicting the survival outcome" as used herein with respect to death by cardiovascular cause, refers to evaluating an individual with CHF and identifying the risk of death from cardiovascular cause. The following describes one embodiment of the method for predicting survival outcome of a patient suffering from CHF.

The patient is first classified as belonging to the group of "Severe" or "Mild to Moderate" CHF. This classification is usually referred to as the "staging of the patient" and should be performed by a trained cardiologist. The basis of the staging is the NYHA functional classification. Patients belonging to the NYHA class III/IV are categorized as suffering from "Severe CHF"; those belonging to the NYHA class I/II are categorized as having "Mild to Moderate CHF". Among patients judged to be borderline between these 2 categories, the examination of the cardiac ejection fraction may be taken into account, a value above 35 - 40 % being more often seen in cases of "Mild to Moderate CHF". The ejection fraction is defined as the ratio of stroke volume to end-diastolic volume (normal values: 67 ± 8 percent). The basal serum or plasma values of BNP, N-proBNP, N-proANP, ET-1 or Big ET-1 can also help in the staging, since they are related to the NYHA classes (see Figure 4, in the examples).

Once the patient is classified as stated above, blood is obtained under appropriate pre-analytical conditions and evaluated for the different natriuretic and vasoactive peptides. The patient should be at rest for 20 - 30 minutes, in a semi-supine position. Blood obtained by venipuncture is collected in accordance with the particular assay to be used.

Each laboratory should establish its own normal values for the natriuretic and vasoactive peptides that will be determined. The normal population on which these values are established should be age and gender matched to the population of cardiac patients seen in their specified setting. Since the distributions of these parameters (or markers) are usually asymmetrical, logarithmic transformation is applied and the normal reference range is expressed as a geometrical mean ± 2SD.

If the patient is clearly classified as belonging to the group of "Severe CHF", the two preferred assays to perform and to interpret in combination are Big ET-1 and N-proANP. The results are expressed as a multiple of the geometrical mean of the normal population (example: Big ET-1 is approximately 4.1 fold and N-proANP is approximately 3.8 fold higher than the geometrical mean of their respective normal values). These values are then compared to cutoff values defined as described herein. If two parameters (or markers) are above their specified cutoff values, the outcome of the patient in his/her category is worst. If both parameters (or markers) are below their specified cutoff values, the outcome of that patient in his/her category is best. If only one of the two parameters (or markers) is above their specified cutoff value, the predicted outcome is intermediate between the two extremes defined above. The difference in median survivals between the group with the worst and the best prognosis may be as great as 5 - 10 fold.

If the patient is clearly classified as belonging to the group of "Mild to Moderate CHF", the two preferred assays to perform and to interpret in combination are the N-proANP together with the BNP. The interpretations are based on the same concepts as described above.

The following examples serve to illustrate the present invention. These examples are in no way intended to limit the scope of the invention.

**Preferably the present invention is defined as in the following definitions:**
1. A method of predicting the survival outcome of an individual suffering from severe congestive heart failure, said method comprising:
   a) determining as the first marker a level of Big ET-1 (1-38) or its immunologically detectable fragments in the individual; and
   b) determining as the second marker a level of proANP or its immunologically detectable fragments, proBNP or its immunologically detectable fragments or ET-1 or its immunologically detectable fragments in the individual, wherein Big ET-1 or its immunologically detectable fragments does not include ET-1 or its immunologically detectable fragments;
      or
      a') determining as the first marker a level of N-ProBNP or its immunologically detectable fragments in the individual; and
      b') determining as the second marker a level of BNP or its immunologically detectable fragments in the individual;
         comparing the level of the first and second markers to cut-off levels of these markers in age matched normal individuals, said cut-off levels distinguishing between high and low survival rate due to cardiovascular cause,
         wherein the likelihood of death from cardiovascular cause is greatest when said individual has first and second marker levels that are both greater than their cutoff levels, the likelihood of death from cardiovascular cause is least when said individual has first and second marker levels that are both less than their cutoff levels, and the likelihood of death from cardiovascular cause is intermediate when said individual has one marker level greater than its cutoff level and the other marker level less than its cutoff level.
2. The method of definition 1 wherein said first marker is Big ET-1 (1-38) or its immunologically detectable fragments and said cutoff level is about 2.4-5.0 fold the normal level, and said second marker is proANP or its immunologically detectable fragments and said cutoff level is about 3.3-12 fold the normal level.
3. The method of definition 2, wherein said first marker is Big ET-1.
4. The method of definition 3, wherein said cutoff level of Big ET-1 (1-38) is about 3.5-5.0 fold the normal level for Big ET-1 (1-38).
5. The method of definition 3, wherein said cutoff level of Big ET-1 (1-38) is about 4.1 fold the normal level for Big ET-1 (1-38).
6. The method of definition 2, wherein said first marker is Big ET-1 (22-38).
7. The method of definition 6, wherein said cutoff level of Big ET-1 (22-38) is about 2.4-2.6 fold the normal level for Big ET-1 (22-38).
8. The method of definition 6, wherein said cutoff level of Big ET-1 (22-38) is about 2.5 fold the normal level for Big ET-1 (22-38).
9. The method of definition 2, wherein said second marker is proANP.
10. The method of definition 2, wherein said second marker is N-proANP (1-98) or its immunologically detectable fragments.
11. The method of definition 10, wherein said cutoff level of N-proANP (1-98) or its immunologically detectable fragments is about 3.3-4.4 fold the normal level for N-proANP (1-98).
12. The method of definition 10, wherein said cutoff level of N-proANP (1-98) or its immunologically detectable fragments is about 3.8 fold the normal level for N-proANP (1-98).
13. The method of definition 10, wherein said second marker is N-proANP (1-25).
14. The method of definition 13, wherein said cutoff level of N-proANP (1-25) is about 9.5-12 fold the normal level for N-proANP (1-25).
15. The method of definition 13, wherein said cutoff level of N-proANP (1-25) is about 10.7 fold the normal level for N-proANP (1-25).
16. The method of definition 10, wherein said N-proANP is N-proANP (68-98).
17. The method of definition 16, wherein said cutoff level of N-proANP (68-98) is about 8.5-10.8 fold the normal level for N-proANP (68-98).
18. The method of definition 16, wherein said cutoff level of N-proANP (68-98) is about 9.6 fold the normal level for N-proANP (68-98).
19. The method of definition 2, wherein when said level of Big ET-1 (1-38) or its immunologically detectable fragments and proANP or its immunologically detectable fragments are both below the cutoff level, the individual's 50% survival for death from cardiovascular cause is at least about 45 months.
20. The method of definition 2, wherein when said level of Big ET-1 (1-38) or its immunologically detectable fragments and proANP or its immunologically detectable fragments are both below the cutoff level, the individual's 50% survival outcome for death by cardiovascular cause is at least about 75 months.
21. The method of definition 12, wherein when said level of Big ET-1 (1-38) or its immunologically detectable fragments and proANP or its immunologically detectable fragments are both below the cutoff level, the individual's 50% survival outcome for death by cardiovascular cause is at least 91 months.
22. The method of definition 2, wherein when said level of Big ET-1 (1-38) or its immunologically detectable fragments and proANP or its immunologically detectable fragments are both higher than the cutoff level, the individual's 50% survival outcome for death by cardiovascular cause is about 5.5 months.
23. The method of definition 1, wherein severe congestive heart cause is defined as NYHA class III-IV.
24. The method of definition 1 wherein said first marker is Big ET-1 (1-38) or its immunologically detectable fragments, and said cutoff level is about 2.4-5.0 fold the normal level and said second marker is proBNP or its immunologically detectable fragments and said cutoff level is about 4.7-89 fold the normal level.
25. The method of definition 24, wherein said first marker is Big ET-1.
26. The method of definition 25, wherein said cutoff level of Big ET-1 (1-38) is about 3.5-5.0 fold the normal level for Big ET-1 (1-38).
27. The method of definition 25, wherein said cutoff level of Big ET-1 (1-38) is about 4.1 fold the normal level for Big ET-1 (1-38).
28. The method of definition 24, wherein said first marker is Big ET-1 (22-38).
29. The method of definition 28, wherein said cutoff level of Big ET-1 (22-38) is about 2.4-2.6 fold the normal level for Big ET-1 (22-38).
30. The method of definition 28, wherein said cutoff level of Big ET-1 (22-38) is about 2.5 fold the normal level for Big ET-1 (22-38).
31. The method of definition 24, wherein said second marker is N-proBNP or its immunologically detectable fragments and said cutoff level of N-proBNP or its immunologically detectable fragments is about 4.7-6.8 fold the normal level for N-proBNP or its immunologically detectable fragments.
32. The method of definition 24, wherein said first marker is N-proBNP or its immunologically detectable fragments and said cutoff level of N-proBNP or its immunologically detectable fragments is about 5.7 fold the normal level for N-proBNP or its immunologically detectable fragments.
33. The method of definition 24, wherein when said level of Big ET-1 (1-38) or its immunologically detectable fragments and proBNP or its immunologically detectable fragments are both less than the cutoff level, the individual's 50% survival outcome for death by cardiovascular cause is at least about 31-61 months.
34. The method of definition 24, wherein when said level of Big ET-1 (1-38) or its immunologically detectable fragments and proBNP or its immunologically detectable fragments are both more than the cutoff level, the individual's 50% survival outcome for death by cardiovascular cause is about 4.5-7.5 months.
35. The method of definition 31, wherein when said level of Big ET-1 (1-38) or its immunologically detectable fragments and N-proBNP or its immunologically detectable fragments are both less than the cutoff level, the individual's 50% survival outcome for death by cardiovascular cause is about 61 months.
36. The method of definition 31, wherein when said level of Big ET-1 (1-38) or its immunologically detectable fragments and N-proBNP or its immunologically detectable fragments are both more than the cutoff level, the individual's 50% survival outcome for death by cardiovascular cause is about 7.5 months.
37. The method of definition 24 wherein said first marker is Big ET-1 (1-38) or its immunologically detectable fragments, and said cutoff level is about 2.4-5.0 fold the normal level and said second marker is BNP or its immunologically detectable fragments and said cutoff level is about 16-89 fold the normal level.
38. The method of definition 37, wherein said first marker is Big ET-1.
39. The method of definition 38, wherein said cutoff level of Big ET-1 (1-38) is about 3.5-5.0 fold the normal level for Big ET-1 (1-38).
40. The method of definition 38, wherein said cutoff level of Big ET-1 (1-38) is about 4.1 fold the normal level for Big ET-1 (1-38).
41. The method of definition 37, wherein said Big ET-1 or its immunologically detectable fragments is Big ET-1 (22-38).
42. The method of definition 41, wherein said cutoff level of Big ET-1 (22-38) is about 2.4-2.6 fold the normal level for Big ET-1 (22-38).
43. The method of definition 41, wherein said cutoff level of Big ET-1 (22-38) is about 2.5 fold the normal level for Big ET-1 (22-38).
44. The method of definition 37, wherein said cutoff level of BNP or its immunologically detectable fragments is about 32-89 fold the normal level for BNP or its immunologically detectable fragments.
45. The method of definition 37, wherein said cutoff level of BNP or its immunologically detectable fragments is about 54 fold the normal level for BNP or its immunologically detectable fragments.
46. The method of definition 37, wherein said cutoff level of BNP or its immunologically detectable fragments is about 16-21 fold the normal level for BNP or its immunologically detectable fragments.
47. The method of definition 37, wherein said cutoff level of BNP or its immunologically detectable fragments is about 18 fold the normal level for BNP or its immunologically detectable fragments.
48. The method of definition 37, wherein when said level of Big ET-1 (1-38) or its immunologically detectable fragments and BNP or its immunologically detectable fragments are both less than the cutoff level, the individual's 50% survival outcome for death by cardiovascular cause is about 31-44 months.
49. The method of definition 37, wherein when said level of Big ET-1 (1-38) or its immunologically detectable fragments and BNP or its immunologically detectable fragments are both more than the cutoff level, the individual's 50% survival outcome from death by cardiovascular cause is about 4.5 months.
50. The method of definition 1 wherein said first marker is Big ET-1 (1-38) or its immunologically detectable fragments, and said cutoff level is about 2.4-5.0 fold the normal level and said second marker is ET-1 or its immunologically detectable fragments and said cutoff level is about 1.9-2.2 fold the normal level.
51. The method of definition 50, wherein said cutoff level of Big ET-1 (1-38) or its immunologically detectable fragments is about 3.5-5.0 fold the normal level for Big ET-1 (1-38) or its immunologically detectable fragments.
52. The method of definition 50, wherein said cutoff level of Big ET-1 (1-38) or its immunologically detectable fragments is about 4.1 fold the normal level for Big ET-1 (1-38) or its immunologically detectable fragments.
53. The method of definition 50, wherein said first marker is Big ET-1 (22-38).
54. The method of definition 52, wherein said cutoff level of Big ET-1 (22-38) is about 2.4-2.6 fold the normal level for Big ET-1 (22-38).
55. The method of definition 53, wherein said cutoff level of Big ET-1 (22-38) is about 2.5 fold the normal level for Big ET-1 (22-38).
56. The method of definition 50, wherein said cutoff level of ET-1 is about 2.1 fold the normal level for ET-1.
57. The method of definition 50, wherein when said level of Big ET-1 (1-38) or its immunologically detectable fragments and ET-1 or its immunologically detectable fragments are both below the cutoff level, the individual's 50% survival outcome for death by cardiovascular cause is about 37 months.
58. The method of definition 50, wherein when said level of Big ET-1 (1-38) or its immunologically detectable fragments and ET-1 or its immunologically detectable fragments are both more than the cutoff level, the individual's 50% survival outcome for death by cardiovascular cause is about 4.5 months.
59. The method of definition 1 wherein said first marker is N-proBNP or its immunologically detectable fragments, and said cutoff level is about 4.7-6.8 fold the normal level and said second marker is BNP or its immunologically detectable fragments and said cutoff level is about 16-89 fold the normal level.
60. The method of definition 59, wherein said cutoff level of N-proBNP or its immunologically detectable fragments is about 5.7 fold the normal level for N-proBNP or its immunologically detectable fragments.
61. The method of definition 59, wherein said cutoff level of BNP or its immunologically detectable fragments is about 32-89 fold the normal level for BNP or its immunologically detectable fragments.
62. The method of definition 59, wherein said cutoff level of BNP or its immunologically detectable fragments is about 54 fold the normal level for BNP or its immunologically detectable fragments.
63. The method of definition 59, wherein said cutoff level of BNP or its immunologically detectable fragments is about 16-21 fold the normal level for BNP or its immunologically detectable fragments.
64. The method of definition 59, wherein said cutoff level of BNP or its immunologically detectable fragments is about 18 fold the normal level for BNP or its immunologically detectable fragments.
65. The method of definition 59, wherein when said level of N-ProBNP or its immunologically detectable fragments and BNP or its immunologically detectable fragments are both less than the cutoff level, the individual's 50% survival outcome from death by cardiovascular cause is about 44 months.
66. The method of definition 59, wherein when said level of N-ProBNP or its immunologically detectable fragments and BNP or its immunologically detectable fragments are both more than the cutoff level, the individual's 50% survival outcome for death by cardiovascular cause is about 5 months.
67. A method of predicting the survival outcome of an individual suffering from mild congestive heart cause, said method comprising:
   a) determining as the first marker a level of proANP or its immunologically detectable fragments in the individual; and
   b) determining as the second marker a level of proBNP or its immunologically detectable fragments or Big ET-1 (1-38) or its immunologically detectable fragments in the individual;
   c) comparing the level of the first and second markers to cut-off levels of these markers in age matched normal individuals, said cut-off levels distinguishing between high and low survival rate,
      wherein the likelihood of death from cardiovascular cause is greatest when said individual has first and second marker levels that are both greater than their cutoff levels, the likelihood of death from cardiovascular cause is least when said individual has first and second marker levels that are both less than their cutoff levels, and the likelihood of death from cardiovascular cause is intermediate when said individual has one marker level greater than its cutoff level and the other marker level less than its cutoff level.
68. The method of definition 67, wherein said first marker is proANP.
69. The method of definition 67, wherein said first marker is N-proANP or its immunologically detectable fragments and said cutoff level is about 1.7-3.3 fold the normal level and said second marker is proBNP or its immunologically detectable fragments and said cutoff level is about 1.3-7 fold the normal level of proBNP or its immunologically detectable fragments.
70. The method of definition 69, wherein N-proANP or its immunologically detectable fragments is N-ProANP (1-98).
71. The method of definition 70, wherein said cutoff level of N-proANP (1-98) is about 1.7-2.2 fold the normal level for N-proANP (1-98).
72. The method of definition 70, wherein said cutoff level of N-proANP (1-98) is about 1.9 fold the normal level for N-proANP (1-98).
73. The method of definition 69, wherein N-proANP or its immunologically detectable fragments is N-ProANP (1-25).
74. The method of definition 73, wherein said cutoff level of N-proANP (1-25) is about 2.6-3.3 fold the normal level for N-proANP (1-25).
75. The method of definition 73, wherein said cutoff level of N-proANP (1-25) is about 3 fold the normal level for N-proANP (1-25).
76. The method of definition 69, wherein N-proANP or its immunologically detectable fragments is N-ProANP (68-98).
77. The method of definition 76, wherein said cutoff level of N-proANP (68-98) is about 2.4-3.1 fold the normal level for N-proANP (68-98).
78. The method of definition 76, wherein said cutoff level of N-proANP (68-98) is about 2.8 fold the normal level for N-proANP (68-98).
79. The method of definition 67, wherein said proBNP or its immunologically detectable fragments is BNP or its immunologically detectable fragments.
80. The method of definition 79, wherein said cutoff level of BNP or its immunologically detectable fragments is 2.6-7 fold the normal level for BNP or its immunologically detectable fragments.
81. The method of definition 79, wherein said cutoff level of BNP or its immunologically detectable fragments is 4.4-5.7 fold the normal level for BNP or its immunologically detectable fragments.
82. The method of definition 67, wherein said proBNP or its immunologically detectable fragments is N-proBNP or its immunologically detectable fragments.
83. The method of definition 82, wherein said cutoff level of N-proBNP or its immunologically detectable fragments is 1.3-1.8 fold the normal level for N-proBNP or its immunologically detectable fragments.
84. The method of definition 82, wherein said cutoff level of N-proBNP or its immunologically detectable fragments is 1.5 fold the normal level for N-proBNP or its immunologically detectable fragments.
85. The method of definition 67, wherein when said level of ProANP or its immunologically detectable fragments and proBNP or its immunologically detectable fragments are both less than the cutoff level, the individual's 75% survival outcome for death by cardiovascular cause is at least about 91 months.
86. The method of definition 67, wherein when said level of ProANP or its immunologically detectable fragments and proBNP or its immunologically detectable fragments are both more than the cutoff level, the individual's 75% survival outcome for death by cardiovascular cause is at about 35-42 months.
85. The method of definition 68, wherein when said level of N-ProANP or its immunologically detectable fragments and BNP or its immunologically detectable fragments are both less than the cutoff level, the individual's 75% survival outcome for death by cardiovascular cause is at least about 91 months.
86. The method of definition 68, wherein when said level of N-ProANP or its immunologically detectable fragments and BNP or its immunologically detectable fragments are both more than the cutoff level, the individual's 75% survival outcome for death by cardiovascular cause is about 35-42 months.
87. The method of definition 68, wherein when said level of N-ProANP or its immunologically detectable fragments and BNP or its immunologically detectable fragments are both more than the cutoff level, the individual's 75% survival outcome for death by cardiovascular cause is about 40 months.
88. The method of definition 67, wherein mild congestive heart cause is defined as NYHA class I-II.
89. The method of definition 67, wherein said first marker is proANP or its immunologically detectable fragments and said cutoff level is about 1.7-3.3 fold the normal level and said second marker is Big ET-1 (1-38) or its immunologically detectable fragments and said cutoff level is about 1.6-2.4 fold the normal level.
90. The method of definition 89, wherein said first marker is proANP.
91. The method of definition 90, wherein said first marker is N-ProANP (1-98).
92. The method of definition 91, wherein said cutoff level of N-proANP (1-98) is about 1.7-2.2 fold the normal level for N-proANP (1-98).
93. The method of definition 91, wherein said cutoff level of N-proANP (1-98) is about 1.9 fold the normal level for N-proANP (1-98).
94. The method of definition 90, wherein said second marker is Big ET-1 (1-38).
95. The method of definition 94, wherein said cutoff level of Big ET-1 (1-38) is about 1.6-2.4 fold the normal level for Big ET-1 (1-38).
96. The method of definition 94, wherein said cutoff level of Big ET-1 (1-38) is about 2 fold the normal level for Big ET-1 (1-38).
97. The method of definition 90, wherein when said level of ProANP or its immunologically detectable fragments and Big ET-1 (1-38) or its immunologically detectable fragments are both less than the cutoff level, the individual's 75% survival outcome for death by cardiovascular cause is at least about 90 months.
98. The method of definition 90, wherein when said level of ProANP or its immunologically detectable fragments and Big ET-1 (1-38) or its immunologically detectable fragments are both more than the cutoff level, the individual's 75% survival outcome for death by cardiovascular cause is about 40 months.

### EXAMPLES

### I. Materials and methods for determining prognosis in congestive heart failure

### A. Patients and study design

Over a 14 month period, a series of 87 patients were prospectively included in a neurohormonal survival study, 47 patients with severe CHF (NYHA class III to IV) optimally treated with angiotensin converting enzyme-inhibitors, furosemide, spironolactone and beta-blockers and 40 patients with mild to moderate CHF (NYHA class I to II). The clinical follow-up was performed by the same cardiologist for an additional seven years. Neurohormonal plasma samples were obtained at entry into the study. To provide control values for neurohormonal markers, blood samples were obtained from 28 - 92 healthy subjects. Each patient gave informed consent and the protocol was approved by the local Institutional Review Board.

### B. Measurements of neurohormonal markers

Venous blood samples were obtained after 30 minutes of rest in the supine position. Blood samples were collected as previously described (⁶)

The levels of plasma ET-1 were determined in this study by standard radioimmunoassay (RIA) after prior extraction and concentration of the sample on Sep-Pack C₁₈ cartridges. The assay used was based on the method previously described (^{6;44}). Blood was drawn in non fasting conditions, at any time of the day, after a 30 minutes recumbence period. Blood was collected in chilled tubes (Sarstedt^{®}) containing EDTA 3 mM.L⁻¹ and benzamidine 9 mM.L⁻¹ The tubes were stored between 1 and 4 hours on wet ice until centrifugation. After centrifugation in a refrigerated centrifuge (1500 g; 15 minutes; 4°C), plasma was separated and frozen at -80°C. After thawing, 3 ml of unfrozen plasma mixed with 2 g of guanidine hypochloride (Sigma; Catalog # G-4505); the mixture was vortexed and incubated at room temperature for 5 - 10 minutes. The mixture was then applied to a Sep-Pack C₁₈ cartridge (Waters, Catalog # Wat051910), previously activated with 8 ml of a solution of acetonitrile containing 0.1 % of trifluroacetic acid (TFA; Sigma, Catalog # T6508). The cartridge was then washed with 8 ml of MilliQ^{®} water containing 0.1 % TFA. Proteins were eluted with 3 ml of a solution of acetonitrile/MilliQ^{®} water (70/30) containing 0.1 % of TFA. The eluate was lyophilized in a "Speedvac concentrator" and the dried pellet was resuspended in the RIA buffer. This solution was kept in polypropylene tubes at -70°C until the assay.

| RIA Assay buffer: | |
|---|---|
| Sodium phosphate dibasic (Na₂HPO₄) (Merck, Catalogue # 6580) | 14.41g |
| Sodium phosphate monobasic (NaH₂PO₄) | 2.62g |
| Sodium Chloride (NaCl) (Sigma, Catalogue # S-9625) | 2.92g |
| Azide (NaN₃) (Merck, Catalogue # 6688) | 0.1g |
| Human albumin (Behring, Catalogue # ORHA A20/C01) | 1g |
| Ethylenediaminetetraaceti acid di-sodium salt (EDTA-Na) | 3.72g |
| Triton X-110 (Fluka, Catalogue # T6878) | 1ml |
| Eau MilliQ^{®} Water | 1L |
| Adjust pH at 7.4 | |

Synthetic ET-1 for iodination and standards was obtained from Peninsula (catalog # 6901).

The RIA was performed with ¹²⁵I-labeled ET-1, obtained after iodination by the lactoperoxidase method and separation of the iodinated peptide from the uniodinated peptide by RP-HPLC. The anti-ET-1 antiserum was obtained from Peninsula (Catalog # RAS6901). This antiserum fully recognized ET-1 (1 - 21), did not cross-react significantly with the Big ET-1 (22 - 38) fragment, but had a 14 % cross-reactivity with the Big ET-1 (1 - 38).

The RIA was performed by incubating increasing concentrations of ET-1 (50 µl) (standard curve) or reconstituted sample extract (50 µl) with the anti-ET-1 antiserum (diluted to obtain a 30% tracer binding at the end of the incubation), for 18 - 24 hours at room temperature (18 - 24 °C); 10,000 cpm of ¹²⁵-I-ET-1 (100 µl) was then added and incubation was continued at room temperature for 5 hours. Bound and free ET-1 were separated by immunoprecipitation with a goat anti-rabbit antiserum. The radioactivity of the pellets was determined in a gamma counter and the concentrations of ET-1 in the samples derived from the standard curve analyzed with a cubic-spline model curve fit. Recoveries, intra-assay and inter-assay coefficients of variation were 68%, 12% and 16%, respectively. Minimal detectable dose was of 2 pg/ml.

For the Big ET-1 (22-38) RIA, blood was collected as described for the ET-1 assay and plasma was extracted and concentrated as for the ET-1 assay. The anti-Big ET-1 anti-serum was prepared by immunization of rabbits with the Big ET-1 (22-38) fragment, coupled to keyhole limpet hemocyanin (KLH). This antiserum fully recognizes the Big ET-1 (22 - 38) fragment, does not cross-react significantly with ET-1 (1 - 21), but has an 18 % cross-reactivity with the Big ET-1 (1 - 38). The RIA was performed according to the same standard procedure as described for the assay of ET-1. Recoveries, intra-assay and interassay coefficients of variation were of 76%, 5% and 8%, respectively. Minimal detectable dose was of 2 pg/ml.

The plasma Big ET-1 (1-38) was determined with a commercial 2-site ELISA assay (Biomedica Gruppe, Wien Austria) and run according to the manufacturer's directional insert. The assay recognizes mostly full-length Big ET-1 (1-38) and demonstrates no significant reactivity with ET (22 - 38) or ET-1. Intra-assay and inter-assay coefficients of variation were lower than 7% and 10% respectively.

The levels ofN-proANP (1-98) were determined with 2 RIAs. Fragments of the amino-terminal fraction and the carboxy-terminal fraction of the N-proANP (1-98) would also be detected by these assays. Blood was collected as described for the ET-1 assay and plasma was extracted and concentrated as for the ET-1 assay. N-proANP (68- 98) antiserum was obtained from Unite de Diabétologie et Nutrition, Département de Médecine Interne, Faculté de Médecine, Universite Catholique de Louvain; B-1200 Brussels; Belgium. The antiserum was prepared by immunizing rabbits with the N-proANP (68 - 98) coupled to keyhole limpet hemocyanin (KLH). This antiserum fully recognized N-pro-ANP (68 - 98), full-length N-proANP (1-98) but did not cross-react significantly with N-proANP (1 -25). Another antiserum against N-proANP (1 - 25) was obtained from Peninsula Labs (Peninsula Laboratories Ltd. Rurope, St. Helens, Merseydide, UK). This antiserum recognized N-proANP (1-25), full-length N-proANP (1-98) but did not cross-react significantly with N-proANP (68-98). The RIAs were performed according to the same standard procedure as described for the assay of ET-1. Mean estimates of recoveries, intra-assay and interassay coefficients of variation for both assays were 65%, 7% and 13%, respectively. Minimal detectable dose was of 30 - 47 pg/ml pg/ml.

The N-proANP (1 - 98) 2 site ELISA was performed using commercial reagents (Biomedica Gruppe, Wien Austria) according to the manufacturer's directional insert. The assay recognized the full-length N-proANP (1 - 98) and presented no significant cross-reactivity with N-proANP (1 -25) or N-pro ANP (68-98) peptides. Intraassay and interassay coefficients of variation were lower than 7% and 10% respectively.

The levels of circulating BNP were measured by RIA. Blood was collected as described for the ET-1 assay and plasma was extracted and concentrated as for the ET-1 assay. Anti-BNP antiserum was obtained from Peninsula Labs Peninsula Laboratories Ltd. Rurope, St. Helens, Merseydide, UK. The RIA was performed according to the same procedure described for assay of ET-1. Recoveries were higher than 70%. Intraassay and interassay coefficients of variation were lower than 7% and 10%, respectively. Minimal detectable dose was 2 pg/ml.

The levels of BNP were also measured by a commercial IRMA assay (Shinogria, Cisbio, France), following strict adherence to the manufacturer's directional insert. Intraassay and interassay coefficients of variation were respectively 9.7 and 11.4 %.

The levels of circulating N-proBNP were measured with a commercially available competitive ELISA (Biomedica Grruppe, Wien Austria). The antiserum in the assay was directed against an epitope at amino acid position 8 to 29. Intraassay and interassay coefficients of variation were lower than 7% and 10% respectively.

### C. Analysis of the data

Data are expressed as percentages for discrete variables, as the mean values SD for normally distributed variables, and as geometric mean and range for log-normally distributed variables (all serum concentrations of neurohormonal peptides). Log-normally distributed variables were log-transformed before statistical analysis. All tests were two-tailed and p<0.05 was considered as statistically significant. The 3 groups of patients (controls, patients with mild to moderate CHF and patients with severe CHF) were compared using ANOVA with F test (⁴⁵). Significant F tests led to a comparison of groups two by two using Student t tests with a Bonferroni corrected p value.

The value of the different markers in predicting survival was evaluated, when they were used alone or in combination. This was done independently for the group of patients with severe (NYHA Class III-IV) or mild to moderate (NYHA Class I-II) congestive heart failure (CHF).

In a first step, a cutoff value was determined for each parameter by trial and error iterations. The cutoff was determined to enable an optimal classification of the patients, into a group with poor prognosis and a group with the better survival prognosis. The survival curves of the groups with values above and below a given cut-off value were estimated using the Kaplan-Meier method. The difference between groups, classified on the basis of each cutoff value, was estimated with the log-rank and the Wilcoxon tests.

In a second step, the cutoff levels determined for each parameter individually were used for a combined classification. For each paired combination, the patients were classified in 3 groups. Group A are patients with two parameters lower than the respective cutoff levels of each parameter; group B, patients with only one of the parameters above the respective cutoff levels of each parameter; group C, patients with both parameters above the respective cutoff levels of each parameter. The estimations of the median survival in each of the 3 subgroups are then analyzed by the Kaplan-Meier method.

All analyses were performed with the JMP Software (Release 5.0.1.2) (SAS Institute Incorporated).

### II. Characteristics of CHF patients studied

The baseline clinical data of CHF patients are summarized in FIG. 1. Most patients were male and had an ischemic cardiomyopathy (87%). Ejection fraction was significantly lower (p<0.001) and age was higher (p<0.01) in patients with severe CHF than in patients with mild to moderate CHF. Of the original group of 47 patients with severe CHF, 34 had died from cardiovascular causes (21 due to worsening heart failure, 11 sudden deaths, 2 due to stroke). One patient underwent emergency heart transplant. Twenty-seven patients (57%) died during the first two years of follow-up. The mean follow-up for the 12 remaining patients was 81±15 months (range:60-91). Of the group of 40 patients with mild to moderate CHF, 15 died (7 sudden deaths, 5 due to worsening heart failure, 1 due to stroke, 1 from aortic aneurysm rupture, and 1 due to pulmonary cancer). The mean follow-up for the 25 remaining patients, was 92±3 months (range:87-96). Survival was significantly lower in patients with severe rather than mild to moderate CHF (p <0.001; FIG. 2). There were significantly more deaths related to worsening HF in the group of patients with severe CHF and there was a trend for more sudden deaths in patients with mild to moderate CHF.

### III. Natriuretic and vasoactive peptides (neurohormonal) measurements: basal values

The normal values of the different neurohormonal parameters measured are represented in FIG. 3.

FIG. 4 shows the baseline levels of the neurohormonal parameters in the groups of patients with mild to moderate and with severe CHF, in comparison to controls. All neurohormonal plasma levels were significantly higher in patients with severe CHF than in healthy subjects. However, only N-proANP 1-98, N-proANP 68-98 and BNP by RIA were significantly higher (p< 0.05) in mild to moderate CHF patients than in healthy control patients. In comparison with the mild to moderate CHF group, all neurohormonal data were significantly increased (p< 0.001) in the severe CHF group.

### IV. The value of different neurohormonal markers in predicting survival in patients with severe CHF (NYHA Class III and IV) used alone or in combination.

FIG. 5 represents the prognostic values for survival using natriuretic and vasoactive peptide assays, considered independently. A cutoff for the 9 different parameters was estimated to optimize the separation of two subgroups of patients, those with the poor and best prognosis. The figure represents the actual concentration of each of the parameters for which the optimal discrimination between the poor and best prognosis groups was obtained. Those concentrations are then expressed as a multiple of the geometrical mean of normals. The figure also shows the cutoff levels expressed as the fold 99.5% confidence limits of the geometrical mean of normals. The median survival times are also represented, with the "test between groups" probability. The most powerful single discriminator between the high and low risk patients (i.e., poor prognosis vs. good prognosis) was the Big ET-1 (1 - 39) 2 site ELISA (P = 0.0005). Big ET-1 (22 - 38), ET-1 (1 -21), N-proANP (1 -98), N-proANP (1 - 25), N-proANP (68 - 98) and N-proBNP (1 -77) assays also provided cutoff values allowing the determination of high and low risk patients, with significantly different (P < 0.05) survival curves. The BNP and BNP (77 -108) assays did not allow statistical discrimination between high risk from low risk patient groups. FIG. 6 and FIG. 7 illustrate the Kaplan-Meier survival curves of the high and low risk patients, when they are determined on the basis of the Big ET-1 (1 - 38) 2 site ELISA or the N-proANP (1 - 98) ELISA, considered alone. FIG. 8 and 9 show the survival curves obtained by using the N-proBNP (competitive ELISA) and the BNP IRMA assays.

In summary, the use of one parameter allowed definition of a poor prognosis group with a median survival time of 9 to 13 months and a good prognosis group with a median survival of 28 to 44 months.

In a second step, Big ET-1 (1 - 38) (or Big ET-1 (22 - 38) was used in combination with the following parameters: N-proANP (1 -98), N-proANP (68 - 98), N-proANP (1 -25), N-proBNP (1 -76), BNP and ET-1. The cutoff levels determined for each parameter individually were used for the combined classification. For each paired combination, the patients were classified in 3 groups. Group A are patients with two parameters lower than their respective cutoff levels of each parameter; group B, patients with only one of the parameters above their respective cutoff levels of each parameter; group C, patients with both parameters above their respective cutoff levels of each parameter. The estimations of the median survival in each group, with the 7 different combinations are shown in FIG. 10. The figure also shows the number of deaths in each group, as well as the number of patients surviving at the end of the study. Wilcoxon tests between groups show significant differences among survival curves for each combination pair tested. The use of Big ET-1 in combination with N-proANP (1 -98), N-proANP (68 - 98), N-proANP (1 -25), N-proBNP (1 -76), BNP or ET-allowed determination of 3 groups of patients: a group of patients with a very poor prognosis of 5.0 to 7.5 months survival; a group of patients with a much better prognosis of 31 to over 61 months of survival; and a group of patients with an intermediate prognosis of 12 to 22 months of survival.

FIG. 11 shows the survival curves of groups A, B and C, classified by using Big ET-1 (1-38) and N-proANP (1-98) in combination. Group C has the worst prognosis, with a median survival time of about 5 months. In strong contrast, group A has a much better prognosis, with a survival which remains slightly above the median.. Group B has a survival prognosis intermediate to groups A and C. FIG. 12 shows that the confidence limits of the survival curves of groups A and C, classified by using the Big ET-1 (1 -38) in combination with N-proANP (1 - 98) do not overlap, a feature strongly in favor of the use of this combination.

The use of Big ET-1(1-38) in combination with N-proANP (1 -98), N-proANP (68 - 98), N-proANP (1 -25), N-proBNP (1 -76), BNP or ET-1 did considerably improve the prediction of poor survival (see FIG. 11), when compared to the predictive value of each parameter considered individually (see FIGs. 6 and 7). Indeed, the combination of 2 parameters allows definition of a group of patients with a median survival time that is about twice that of the high risk group determined with a single test (about 5 months versus 10 months). In addition the combination of Big ET-1 (1-38) with N-proANP (1-98) also allows the identification of a group with an exceptional good prognosis (over 91 months of median survival).

N-proBNP was also used in combination with the BNP. The cutoff levels determined for each parameter individually were used for the combined classification. For each paired combination, the patients were classified in 3 groups. Group A are patients with both parameters lower than their respective cutoff levels; group B, patients with only one of the parameters above its cutoff level; group C, patients with both parameters above their respective cutoff levels. The estimations of the median survival in each group, with the 2 different combinations are shown in FIG. 13. The figure also shows the number of deaths in each group, as well as the number of patients surviving at the end of the study. Wilcoxon tests between groups show significant differences among survival curves for each combination pair tested. The combination of BNP assays (BNP RIA or BNP IRMA) with N-proBNP (1 -76)) resulted in the definition of a group of patients with a very poor prognosis (5 months median survival), in contrast with a group with a much better prognosis (44 months median survival).

FIG. 14 represents the survival of a high and low risk group, determined with N-proBNP (1 - 76) and BNP. The combination of BNP to N-proBNP considerably improved the prognostic power of either parameter considered separately (compare to FIGs. 8 and 9). The survival curves of groups A and C from FIG. 14 are shown with their confidence limits in FIG. 15. A slight overlap of confidence limits is observed.

### V. The value of different neurohormonal markers in predicting survival in patients with mild to moderate CHF (NYHA Class I and II) used alone or in combination.

The value of different neurohormonal markers in predicting survival in a clinically homogeneous group of patients with NYHA I and II class ( mild pathological conditions) was evaluated, when they are used alone or in combination..

FIG. 16 is a table showing the prognostic values for survival using natriuretic and vasoactive peptide assays, considered independently. A cutoff for the 9 different parameters was estimated to optimize the separation of this group into two subgroups, patients with poor and good prognosis. This figure represents the actual concentration of each of the parameters for which the optimal discrimination between the poor from good prognosis groups is obtained. Those concentrations are then expressed as a multiple of the geometrical mean of normals. The figure also shows the cutoff levels expressed as the fold 99.5% confidence limits of the geometrical mean of normals. The 75% survival times estimations are also represented, with the "test between groups" probability. The most powerful single discriminator between the high and low risk patients with mild CHF (NYHA Class I - II) was the N-proANP (1-98) as determined with a 2-site ELISA, followed by the BNP RIA and then the Big ET-1 (1-38) 2-site ELISA. N-proANP (1-25) RIA, N-pro ANP (68-98) RIA, and BNP IRMA, did provide cutoff values allowing the determination of high and low risk patients, but the difference in survival curves was at the limit of significance (P value between 0.05 and 0.10). ET-1 RIA and Big ET-1 (22-38) RIA did not significantly contribute to the prediction of survival in patients with mild to moderate CHF.

FIGs. 17 and 18 illustrate the Kaplan-Meier survival curves of the high and low risk patients, when they are determined on the basis of the N-proANP (1-98) ELISA and the N-proBNP competitive ELISA, considered alone. FIG. 19 shows the survival curves obtained by using the BNP (RIA) alone.

In summary, the use of one marker allowed definition of a poor prognosis group with a 75 % survival time of 39 to 47 months and a good prognosis group with a 75 % survival time exceeding 91 months.

In a second step, N-proANP (1-98) was used in combination with BNP, N-proBNP or Big ET-1 (1-38) to evaluate the survival probability of the patients. The cutoff levels determined for each marker individually were used for the combined classification. For each paired combination, the patients were classified into 3 groups. Group A are patients with two markers lower than their respective cutoff levels of each marker; group B, patients with only one of the markers above their respective cutoff levels of each parameter; group C, patients with both markers above their respective cutoff levels of each parameter. The results are shown in FIG. 20, with the 75% survival estimations, as well as the number of patients who died and survived at the end of the observation period. FIGs. 21 and 22 show the Kaplan-Meier survival curves for the groups A, B and C, with two of the combinations [N-proANP (1-98) with N-proBNP, and N-proANP (1-98) with BNP]. With both combinations, group A (two markers above the predefined cutoff) has a much better survival expectation than group C (patients with both markers above the cutoff level).

### VI. References

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising," "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Other embodiments are set forth within the following claims.

## Claims

1. A method of predicting the survival outcome of an individual suffering from mild congestive heart failure, wherein mild congestive heart failure is defined as NYHA class I-II, said method comprising:
a) determining as the first marker a level ofN-proANP or its immunologically detectable fragments in the individual; and
b) determining as the second marker a level of BNP or its immunologically detectable fragments in the individual;
c) comparing the level of the first and second markers to cut-off levels of these markers in age matched normal individuals, said cut-off levels distinguishing between high and low survival rate,
wherein the likelihood of death from cardiovascular cause is greatest when said individual has first and second marker levels that are both greater than their cutoff levels, the likelihood of death from cardiovascular cause is least when said individual has first and second marker levels that are both less than their cutoff levels, and the likelihood of death from cardiovascular cause is intermediate when said individual has one marker level greater than its cutoff level and the other marker level less than its cutoff level.

2. The method of claim 1, wherein said first marker is N-proANP or its immunologically detectable fragments and said cutoff level is about 1.7-3.3 fold the normal level and said second marker is BNP or its immunologically detectable fragments and said cutoff level is about 2,6-7 fold the normal level of proBNP or its immunologically detectable fragments.

3. The method of claim 2, wherein N-proANP or its immunologically detectable fragments is N-proANP (1-98), N-proANP (1-25) and/or N-proANP (68-98).

4. The method of claim 3, wherein said cutoff level of N-proANP (1-98) is about 1.7-2.2 fold, preferably about 1.9 fold, the normal level for N-proANP (1-98).

5. The method of claim 3, wherein said cutoff level of N-proANP (1-25) is about 2.6-3.3 fold, preferably about 3 fold, the normal level for N-proANP (1-25).

6. The method of claim 3, wherein said cutoff level of N-proANP (68-98) is about 2.4-3.1 fold, preferably about 2.8 fold, the normal level for N-proANP (68-98).

7. The method of claim 3, wherein said cutoff level of BNP or its immunologically detectable fragments is 2.6-7 fold, preferably 4.4-5.7 fold, the normal level for BNP or its immunologically detectable fragments.

8. The method of any one of claims 1 to 7, wherein when said level of N-ProANP or its immunologically detectable fragments and BNP or its immunologically detectable fragments are both less than the cutoff level, the individual's 75% survival outcome for death by cardiovascular cause is at least about 91 months.

9. The method of any one of claims 1 to 7, wherein when said level of N-ProANP or its immunologically detectable fragments and BNP or its immunologically detectable fragments are both more than the cutoff level, the individual's 75% survival outcome for death by cardiovascular cause is about 35-42 months.

10. The method of any one of claims 1 to 7, wherein when said level of N-ProANP or its immunologically detectable fragments and BNP or its immunologically detectable fragments are both more than the cutoff level, the individual's 75% survival outcome for death by cardiovascular cause is about 40 months.
